# EUROPÄISCHE PATENTSCHRIFT

(11) **EP 2 041 161 B1**
(45) Veröffentlichungstag und Bekanntmachung des Hinweises auf die Patenterteilung: **18.05.2011**
(21) Anmeldenummer: 07786231.6
(22) Anmeldetag: 20.07.2007
(51) Int. Cl.: C07K 14/00, A61K 38/04

(54) **CHIRALE MIT PHOSPHONSÄUREESTER- ODER PHOSPHONSÄURE- SUBSTITUIERTE VERBINDUNGEN**
CHIRAL COMPOUNDS SUBSTITUTED WITH PHOSPHONATE ESTER OR PHOSPHONIC ACID
COMPOSÉS CHIRAUX SUBSTITUÉS D'ESTER D'ACIDE PHOSPHONIQUE OU D'ACIDE PHOSPHONIQUE

(30) Priorität: 21.07.2006 DE 102006034319
(43) Veröffentlichungstag der Anmeldung: 01.04.2009
(73) Patentinhaber: Ugichem GmbH, 6020 Innsbruck (AT)
(72) Erfinder: LINDHORST, Thomas, 6020 Innsbruck (AT); WERNER, Birgit, 6020 Innsbruck (AT); BOCK, Holger, 6051 Götzens (AT)
(74) Vertreter: Forstmeyer, Dietmar
(86) Internationale Anmeldenummer: PCT/EP2007/006483
(87) Internationale Veröffentlichungsnummer: WO 2008/009470

(56) Entgegenhaltungen:
- WO-A-00/52038
- WO-A-95/16202

## Beschreibung

Die vorliegende Erfindung betrifft neue Verbindungen, die mit Phosphonsäureester- oder Phosphonsäure-Funktionen. substituierte PNA-Einheiten enthalten und mindestens zwei chirale zentren aufweisen.

Nach der Erstinfektion einer Wirtszelle durch einen HI-Virus zeigen die bekannten antiviralen Verbindungen (z.B. Indinavir) eine Wirkung nur auf die unmittelbare erste virale Tochtergeneration, indem sie den Replikationszyklus unterbrechen. Dies führt zu einer messbaren Verringerung der Virenanzahl gegenüber unbehandelten Wirtszellen. Diese Reduzierung der Viren geschieht allerdings nicht zu 100%. Isoliert man überlebende Viren, so sind sie weiterhin in der Lage, vorher nicht infizierte Wirtszellen zu infizieren und einen vollständigen Replikationszyklus zu durchlaufen.

PNAs (Peptid-Nukleinsäuren) sind synthetische DNA/RNA-Analoga mit einem N-(2-Aminoethyl)glycin-Gerüst (NB = Nukleobase, n = 0-50; R¹, K, L = Substituenten). Sie werden durch Knüpfen von Peptidbindungen zwischen N-Acetyl-N-(2-aminoethyl)glycin-Bausteinen (PNA-Monomere) hergestellt. Jeder einzelne dieser N-Acetyl-N-(2-aminoethyl)glycin-Bausteine stellt eine PNA-Einheit dar.

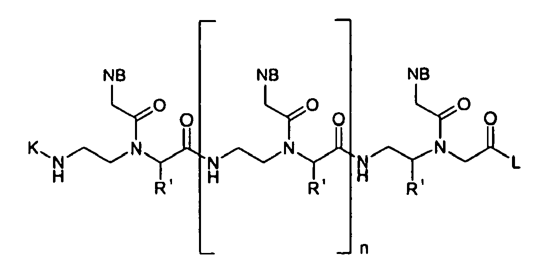

PNAs sind unter physiologischen Bedingungen resistent gegen hydrolytische (enzymatische) Spaltung. Es ist bekannt, dass PNAs komplementäre Nukleinsäuresequenzen (DNA oder RNA) Sequenz-spezifisch erkennen und an diese mit höherer Affinität als ihre natürlichen Vorbilder binden können (M. Egholm, O. Buchardt, L. Christensen, C. Behrens, S.M. Freier, D.A. Driver, R.H. Berg, S.K. Kim, B. Norden, P.E. Nielsen, Nature, 1993, 365, 566-568. B. Hyrup, P.E. Nielsen, Bioorg. Med. Chem., 1996, 4, 5-23).

PNAs werden zum Beispiel als Antisense-Oligomere eingesetzt. Dabei wird durch Hybridisierung eines Antisense-Oligomers an die Protein-spezifische mRNA die Expression des Proteins auf der Ebene der Translation inhibiert. Aufgrund dieser Eigenschaften sind PNAs geeignete Verbindungen für die Verwendung z.B. als Diagnostika.

Bekannte PNA-Moleküle haben den Nachteil, dass sie, verglichen mit DNA, schwer wasserlöslich sind. Ferner ist das Durchqueren der Zellmembran ein allgemeines Problem für PNAs, wodurch die Aufnahme in Zellen nur äußerst langsam erfolgt.

Aus US 5719262 sind PNAs bekannt, bei denen durch AminFunktionen an dem Rest R¹ die Wasserlöslichkeit gesteigert werden konnte. Aber auch derartig modifizierte PNAs besitzen nach wie vor eine schlechte Zellgängigkeit. Dadurch ist die Verwendung von PNAs als Antisense-Wirkstoffe in lebenden Organismen stark limitiert.

Aus EP 1157031 sind Oligomere bekannt, die ein oder mehrere Phosphonsäureester- oder Phosphonsäure-Funktionen aufweisen. Derart modifizierte Oligomere sind besser zellgängig im Vergleich zu PNAs, die diese Substituenten nicht enthalten.

Allerdings ist eine gute Zellgängigkeit von Antisense-Oligomeren allein nicht ausreichend, um in biologischen Systemen einen starken Effekt der Unterdrückung der Genexpression zu erzielen.

Aufgabe der vorliegenden Erfindung ist es daher, Verbindungen bereitzustellen, die in der Lage sind, nicht nur die Virenanzahl der ersten Tochtergeneration zu verringern, sondern die darüber hinaus auch einen verstärkt reduzierenden Effekt auf die Virenzahl der zweiten Tochtergeneration aufweisen, sowie deren Anwendungen. Dadurch erhält man eine Substanzklasse, die in Lage ist, über zwei Virengenerationen eine Wirksamkeit zu zeigen.

Diese Aufgabe wird gelöst durch Verbindungen der allgemeinen Formel I: 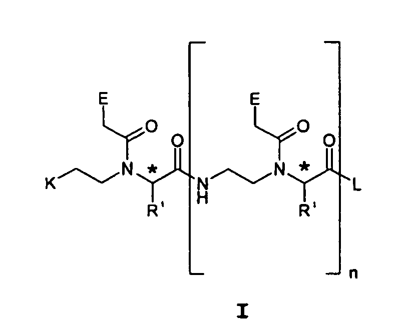 worin
n eine ganze Zahl von 7 bis 35 ist, bevorzugt 9 bis 28, am stärksten bevorzugt 13 bis 20.
E ist unabhängig voneinander ein H-Atom, ein substituierter oder unsubstituierter Phenylrest, ein substituierter oder unsubstituierter Heterocyclus, eine gegebenenfalls mit Schutzgruppen substituierte Nukleobase, z.B. eine in der Natur vorkommende oder nicht in der Natur vorkommende Nukleobase, oder ein DNA-Intercalator.

Bevorzugt ist jedes E unabhängig voneinander ein Adeninyl-, Cytosinyl-, Pseudoisocytosinyl-, Guaninyl-, Thyminyl-, Uracilyl- oder Phenyl-Rest.

Jeder Rest R¹ ist unabhängig voneinander ein H-Atom oder ein gegebenenfalls substituierter Alkyl-, Alkenyl-, Alkylaryl-, Aryl- oder alicyclischer Rest mit bis zu 20 C-Atomen, wobei mindestens ein Rest R¹ kein H-Atom und mit einer oder mehreren Phosphonsäureester- oder Phosphonsäure-Funktionen substituiert ist.

Wenn der Rest R¹ nicht mit einer oder mehreren Phosphonsäureester- oder Phosphonsäure-Funktionen substituiert ist, kann er z.B. auch unabhängig voneinander eine oder mehrere Seitenketten einer natürlichen oder nichtnatürlichen Aminosäure aufweisen, bevorzugt einen gegebenenfalls substituierten Alkyl-, Alkenyl-, Alkylaryl-, Aryl-, heterocyclischen oder alicyclischen Rest mit bis zu 20 C-Atomen.

Bevorzugt umfasst jeder Rest R¹ unabhängig voneinander 1, 2, 3, 4, 5, 6, 7, 8, 9 oder 10 C-Atome.

Jeder Rest R¹ kann unabhängig voneinander verzweigt oder unverzweigt sein.

Der Ausdruck "gegebenenfalls substituiert" bezieht sich auf Gruppen, in denen ein oder mehrere Wasserstoffatome durch Fluor-, Chlor-, Brom- oder Jodatome oder -COOH, COOR⁸, -CSOH, CSOR⁸, -COSH, COSR⁸, -CONH₂, -CONHR⁹, -COR¹⁰R¹¹, -OH, -OR⁸, =O, - SH, -SR⁸, =S, -NH₂, =NH, -NHR⁹, -NR¹⁰R¹¹, -NR¹²NOH, -NOR¹³, NO₂-Gruppen, Phosphonsäureester- oder Phosphonsäure-Funktionen ersetzt sind. Dieser Ausdruck bezieht sich weiterhin auf Gruppen, die mit unsubstituierten C₁-C₆ Alkyl-, C₂-C₆ Alkenyl-, C₂-C₆ Alkinyl-, C₁-C₆ Heteroalkyl, C₃-C₁₀ Cycloalkyl-, C₂-C₉ Heterocycloalkyl-, C₆-C₁₀ Aryl-, C₅-C₉ Heteroaryl, C₇-C₁₂ Aralkyl- oder C₂-C₁₁ Heteroaralkyl-Gruppen substituiert sind, wobei R⁸, R⁹, R¹⁰, R¹¹, R¹² und R¹³ unabhängig voneinander C₁-C₆ Alkylreste sind.

Phosphonsäureester-Funktionen können zum Beispiel die Formel -P(=O)(OV)₂ oder -P(=O)(OV)(OH) aufweisen. Dabei kann jedes V unabhängig voneinander ein unsubstituierter Alkyl-, Alkenyl-, Alkylaryl-, Aryl-, oder alicyclischer Rest mit bis zu 20 C-Atomen, stärker bevorzugt mit bis 7 C-Atomen und am stärksten bevorzugt ein Methyl-, Ethyl-, Cyclohexyl, oder Benzyl-Rest sein.

Bei den erfindungsgemäßen Verbindungen können die Phosphonsäure-Funktionen zum Beispiel die Formel -P(=O)(OH)₂ aufweisen.

Am stärksten bevorzugt wird jeder Rest R¹ unabhängig voneinander aus einer Gruppe der Formeln -(C1-C10)Alkyl-[P(=O) (O-V)₂] ausgewählt, wobei jedes V unabhängig voneinander ein H-Atom, ein Methyl-, Ethyl-, Cyclohexyl-, oder ein Benzyl-Rest ist.

K ist eine Gruppe der Formel -NR²R³, -N^{⊕}R²R³R⁴, -NR² (CO) R³ oder - NR² (CS) R³, wobei R², R³ und R⁴ unabhängig voneinander ein H-Atom, ein Alkylrest, Amino-Schutzgruppe, Reporterligand, Fluoreszenz-Marker, Intercalator, Chelator, Aminosäure, Peptid, Protein, Kohlenhydrat, Lipid, Steroid, Fettsäure, Oligonukleotid, Quantum-Dot, FRET-Quencher (Fluoreszenz-Resonanz-Energie-Transfer-Quencher) oder ein in Wasser lösliches oder unlösliches Polymer sind, wobei jeder der vorstehenden Reste gegebenenfalls substituiert sein kann.

Bevorzugt ist K eine NH₂-Funktion, ein -NH(CO)CH₃-Rest, eine Gruppe der Formel -NR²R³ oder -N^{®}R²R³R⁴ oder -NR²(CO)R³, wobei R², R³ und R⁴ unabhängig voneinander ein H-Atom, ein(e) unsubstituierte(r) oder mit Phosphonsäureester- oder Phosphonsäure-Funktionen substituierte(r) Aminosäure, Peptid oder Alkylrest sind, wobei jeder der vorstehenden Reste gegebenenfalls substituiert sein kann.

L ist eine Gruppe der Formel -NR⁵R⁶, -NR⁵(CO)R⁶, -NR⁵(CS)R⁶, -OR⁷ oder -SR⁷, wobei R⁵ und R⁶ unabhängig voneinander ein H-Atom, ein Alkylrest, Reporterligand, Fluoreszenz-Marker, Intercalator, Chelator, Aminosäure, Aminosäureamid, Peptid, Peptidamid, Protein, Kohlenhydrat, Lipid, Steroid, Fettsäure, Oligonukleotid, Quantum-Dot, FRET-Quencher oder ein in Wasser lösliches oder unlösliches Polymer sind, wobei jeder der vorstehenden Reste gegebenenfalls substituiert sein kann und R⁷ ein H-Atom ein Alkylrest, Reporterligand, Fluoreszenz-Marker, Intercalator, Chelator, Aminosäure, Aminosäureamid, Peptid, Peptidamid, Protein, Kohlenhydrat, Lipid, Steroid, Fettsäure, Oligonukleotid, Quantum-Dot, FRET-Quencher oder ein in Wasser lösliches oder unlösliches Polymer ist, wobei jeder der vorstehenden Reste gegebenenfalls substituiert sein kann.

Bevorzugt ist L eine OH-Funktion, eine NH₂-Funktion, eine -NH-(C₁-C₅)Alkyl-Funktion, eine unsubstituierte oder mit Phosphonsäureester- oder Phosphonsäure-Funktionen substituierte Aminosäure-, Aminosäureamid-, Peptid- oder Peptidamid-Einheit, wobei jeder der vorstehenden Reste gegebenenfalls substituiert sein kann.

In der gesamten Anmeldung können Alkylreste vorzugsweise 1-6 C-Atome aufweisen, z. B. Methyl-, Ethyl-, Propyl- oder Butyl-Gruppen sein.

Wenn R¹ kein H-Atom ist, entsteht durch die Bindung vom Rest R¹ an das Backbone der allgemeinen Verbindung **I** an der Verbindungsstelle ein asymmetrisches Zentrum (*). An jedem asymmetrischen Zentrum liegt demnach ein R-Konfiguration oder eine S-Konfiguration vor.

Dabei wird die Konfiguration vorzugsweise am asymmetrischen Zentrum analog den Cahn-Ingold-Prelog-Regeln definiert, mit der zusätzlichen Maßgabe, dass die Priorität der Liganden immer wie folgt definiert ist: Das Stickstoffatom am asymmetrischen Zentrum erhält immer die Priorität 1. Das Kohlenstoffatom der Carboxylgruppe am asymmetrischen Zentrum erhält immer die Priorität 2. Das Kohlenstoffatom des Rests R¹ am asymmetrischen Zentrum erhält immer die Priorität 3. Das Wasserstoffatom am asymmetrischen Zentrum erhält immer die Priorität 4.

In EP 1157031 werden Oligomere beschrieben, die ausschließlich aus racemischen, mit Phosphonsäureester- oder Phosphonsäure-Funktionen substituierten Monomeren hergestellt werden. Beispielsweise existieren bei einem aus 15 racemischen Monomeren aufgebauten Oligomer 2¹⁵ verschiedene Kombinationen der Stereozentren (*) oder 32768 verschiedene Stereoisomere. Man erhält dort ein Gemisch aus Verbindungen mit unterschiedlichen chemischen und physikalischen Eigenschaften.

Im Gegensatz zu den in EP 1157031 beschriebenen Verbindungen werden die hier beschriebenen, erfindungsgemäßen Verbindungen vorzugsweise mit enantiomerenreinen, vorzugsweise mit einer oder mehreren Phosphonsäureester- oder Phosphonsäure-Funktionen substituierten Monomeren hergestellt.

Erfindungsgemäß weisen die Verbindungen der allgemeinen Formel I mindestens 2 asymmetrische Zentren auf, und mindestens 70% der Anzahl der asymmetrischen Zentren, die Reste mit einer oder mehreren Phosphonsäureester- oder Phosphonsäure-Funktionen aufweisen, haben die R-Konfiguration oder die S-Konfiguration, wenn in der Verbindung der allgemeinen Formel I 2 bis 36 asymmetrische Zentren und 1 bis 36 gegebenenfalls substitutierte Reste R¹ mit einer oder mehreren Phosphonsäureester- oder Phosphonsäure-Funktionen vorhanden sind.

Gemäß einer weiteren bevorzugten Ausführungsform der Erfindung entspricht jeder zweite Rest R¹ unabhängig voneinander der Seitenkette einer natürlichen oder nichtnatürlichen Aminosäure, bevorzugt einem gegebenenfalls substituierten Alkyl-, Alkenyl-, Alkylaryl-, Aryl-, heterocyclischen oder alicyclischen Rest mit bis zu 20 C-Atomen und mindestens ein Rest R¹ ist ein gegebenenfalls substituierter Alkyl-, Alkenyl-, Alkylaryl-, Aryl- oder alicyclischer Rest mit bis zu 20 C-Atomen, der mit einer oder mehreren Phosphonsäureester- oder Phosphonsäure-Funktionen substituiert ist, wobei die übrigen Reste R¹ H-Atome sind.

Gemäß einer weiteren bevorzugten Ausführungsform der Erfindung entspricht jeder dritte Rest R¹ unabhängig voneinander der Seitenkette einer natürlichen oder nichtnatürlichen Aminosäure, bevorzugt einem gegebenenfalls substituierten Alkyl-, Alkenyl-, Alkylaryl-, Aryl-, heterocyclischen oder alicyclischen Rest mit bis zu 20 C-Atomen und mindestens ein Rest R¹ ist ein gegebenenfalls substituierter Alkyl-, Alkenyl-, Alkylaryl-, Aryl- oder alicyclischer Rest mit bis zu 20 C-Atomen und mit einer oder mehreren Phosphonsäureester- oder Phosphonsäure-Funktionen substituiert, wobei die übrigen Reste R¹ H-Atome sind.

Gemäß einer weiteren bevorzugten Ausführungsform der Erfindung entsprechen zwei, drei oder mehr benachbarte Reste R¹ unabhängig voneinander der Seitenkette einer natürlichen oder nichtnatürlichen Aminosäure, bevorzugt einem gegebenenfalls substituierten Alkyl-, Alkenyl-, Alkylaryl-, Aryl-, heterocyclischen oder alicyclischen Rest mit bis zu 20 C-Atomen und mindestens ein Rest R¹ ist ein gegebenenfalls substituierter Alkyl-, Alkenyl-, Alkylaryl-, Aryl- oder alicyclischer Rest mit bis zu 20 C-Atomen und mit einer oder mehreren Phosphonsäureester- oder Phosphonsäure-Funktionen substituiert, wobei die übrigen Reste R¹ H-Atome sind.

Gemäß einer weiteren bevorzugten Ausführungsform der Erfindung entspricht jedes R¹ unabhängig voneinander der Seitenkette einer natürlichen oder nichtnatürlichen Aminosäure, bevorzugt einem gegebenenfalls substituierten Alkyl-, Alkenyl-, Alkylaryl-, Aryl-, heterocyclischen oder alicyclischen Rest mit bis zu 20 C-Atomen und mindestens ein Rest R¹ ist ein gegebenenfalls substituierter Alkyl-, Alkenyl-, Alkylaryl-, Aryl- oder alicyclischer Rest mit bis zu 20 C-Atomen und mit einer oder mehreren Phosphonsäureester- oder Phosphonsäure-Funktionen substituiert.

Gemäß einer weiteren bevorzugten Ausführungsform der Erfindung weisen einer oder mehrere der Reste R¹ unabhängig voneinander mindestens eine Phosphonsäureester- oder Phosphonsäure-Funktion auf.

Gemäß weiterer bevorzugter Ausführungsformen der vorliegenden Erfindung gilt folgendes:
1. Sind in der Verbindung der allgemeinen Formel I 2 bis 8 asymmetrische Zentren und 1 bis 8 gegebenenfalls substitutierte Reste R¹ mit einer oder mehreren Phosphonsäureester- oder Phosphonsäure-Funktionen vorhanden, haben mindestens 75% der Anzahl der asymmetrischen Zentren, die Reste mit einer oder mehreren Phosphonsäureester- oder Phosphonsäure-Funktionen aufweisen, die R-Konfiguration, bevorzugt 80%, stärker bevorzugt 85%, stärker bevorzugt 90%, stärker bevorzugt 95%, am stärksten bevorzugt 100%.
2. Sind in der Verbindung der allgemeinen Formel I 9 bis 36 asymmetrische Zentren und 1 bis 36 gegebenenfalls substitutierte Reste R¹ mit einer oder mehreren Phosphonsäureester- oder Phosphonsäure-Funktionen vorhanden, haben mindestens 75% der Anzahl der asymmetrischen Zentren, die Reste mit einer oder mehreren Phosphonsäureester- oder Phosphonsäure-Funktionen aufweisen, die R-Konfiguration, bevorzugt 80%, stärker bevorzugt 85%, stärker bevorzugt 90%, stärker bevorzugt 95%, am stärksten bevorzugt 100%.

Gemäß alternativer bevorzugter Ausführungsformen der vorliegenden Erfindung gilt folgendes:
1. Sind in der Verbindung der allgemeinen Formel I 2 bis 8 asymmetrische Zentren und 1 bis 8 gegebenenfalls substitutierte Reste R¹ mit einer oder mehreren Phosphonsäureester- oder Phosphonsäure-Funktionen vorhanden, haben mindestens 75% der Anzahl der asymmetrischen Zentren, die Reste mit einer oder mehreren Phosphonsäureester- oder Phosphonsäure-Funktionen aufweisen, die S-Konfiguration, bevorzugt 80%, stärker bevorzugt 85%, stärker bevorzugt 90%, stärker bevorzugt 95%, am stärksten bevorzugt 100%.
2. Sind in der Verbindung der allgemeinen Formel **I** 9 bis 36 asymmetrische Zentren und 1 bis 36 gegebenenfalls substitutierte Reste R¹ mit einer oder mehreren Phosphonsäureester- oder Phosphonsäure-Funktionen vorhanden, haben mindestens 75% der Anzahl der asymmetrischen Zentren, die Reste mit einer oder mehreren Phosphonsäureester- oder Phosphonsäure-Funktionen aufweisen, die S-Konfiguration, bevorzugt 80%, stärker bevorzugt 85%, stärker bevorzugt 90%, stärker bevorzugt 95%, am stärksten bevorzugt 100%.

In einer weiteren Ausführungsform sind maximal 50% der Anzahl der Reste R¹ mit Phosphonsäureester- oder Phosphonsäure-Funktionen substituiert und die übrigen Reste R¹ sind H-Atome. In einer weiteren Ausführungsform sind maximal 40% der Anzahl der Reste R¹ mit Phosphonsäureester- oder Phosphonsäure-Funktionen substituiert und die übrigen Reste R¹ sind H-Atome. In einer weiteren Ausführungsform sind maximal 30% der Anzahl der Reste R¹ mit Phosphonsäureester- oder Phosphonsäure-Funktionen substituiert und die übrigen Reste R¹ sind H-Atome. In einer weiteren Ausführungsform sind maximal 20% der Anzahl der Reste R¹ mit Phosphonsäureester- oder Phosphonsäure-Funktionen substituiert und die übrigen Reste R¹ sind H-Atome. In einer weiteren Ausführungsform sind maximal 10% der Anzahl der Reste R¹ mit Phosphonsäureester- oder Phosphonsäure-Funktionen substituiert und die übrigen Reste R¹ sind H-Atome. In einer weiteren Ausführungsform sind maximal 4% der Anzahl der Reste R¹ mit Phosphonsäureester- oder Phosphonsäure-Funktionen substituiert und die übrigen Reste R¹ sind H-Atome.

In einer weiteren bevorzugten Ausführungsform der Erfindung weisen alle asymmetrischen Zentren (*) der allgemeinen Verbindung I die gleiche Konfiguration auf.

In einer weiteren bevorzugten Ausführungsform der Erfindung weisen alle asymmetrischen Zentren (*) der allgemeinen Verbindung **I** die S-Konfiguration auf.

In einer weiteren bevorzugten Ausführungsform der Erfindung weisen alle asymmetrischen Zentren (*) der allgemeinen Verbindung **I** die R-Konfiguration auf.

Ferner werden erfindungsgemäß Zusammensetzungen offenbart, die eine oder mehrere erfindungsgemäße Verbindungen gegebenenfalls in Kombination mit üblichen Adjuvantien enthalten.

Die Synthese der Verbindungen der allgemeinen Formel **I** erfolgt vorzugsweise aus enantiomerenreinen Monomeren. Während der Synthese der Verbindungen der allgemeinen Formel **I** können einzelne asymmetrische Zentren aufgrund der chemischen Synthesebedingungen zu einem geringen Prozentsatz ihre vorher definierte Konfiguration ändern. Der größte Prozentsatz, der während der Synthese gebildeten Verbindungen der allgemeinen Formel I, ist aber stereoisomerenrein. Auch diese Zusammensetzungen sind in der Lage, die erfindungsgemäße Aufgabe zu erfüllen.

Eine Verbindung der allgemeinen Formel **I** kann über die Reste K und L als Linker mit einer zweiten Verbindung der allgemeinen Formel **I** verbunden sein, wobei die Reste wie oben definert sind. Die Konfiguration an den asymmetrischen Zentren der einen Verbindung der allgemeinen Formel **I** ist dabei unabhängig von der Konfiguration an den asymmetrischen Zentren der über den Linker verbundenen zweiten Verbindung der allgemeinen Formel **I.** So können beispielsweise alle asymmetrischen Zentren der einen Verbindung der allgemeinen Formel **I** die R-Konfiguration aufweisen und alle asymmetrischen Zentren der zweiten, verbundenen Verbindung der allgemeinen Formel **I** die S-Konfiguration aufweisen. Es können auch beispielsweise alle asymmetrischen Zentren der einen Verbindung der allgemeinen Formel **I** die R-Konfiguration aufweisen und alle ale asymmetrischen Zentren der zweiten, verbundenen Verbindung der allgemeinen Formel **I** die R-Konfiguration aufweisen.

Der Linker dient inbesondere dazu, den Abstand zwischen zwei Verbindungen der allgemeinen Formel I so einzustellen, dass zwischen den beiden Verbindungen der allgemeinen Formel I mit Linker und einzelsträngiger RNA oder DNA bzw. doppelsträngiger DNA eine gegenseitige Interaktion über die jeweiligen Nukleobasen erfolgen kann.

Als Linker eignen sich alle bekannten und alle für diesen Zweck eingesetzten bzw. einsetzbaren Linkermoleküle. Beispielsweise kann ein solcher Linker eine gegebenenfalls substituierte Alkylkette, ein Peptid, ein Oligonukleotid, oder ein Oligomer, das aus mindestens drei 8-Amino-3,6-dioxaoctansäure-Einheiten (eg1-Einheiten) aufgebaut ist, sein.

Die Substitution mit einer Phosphonsäureester- bzw. Phosphonsäure-Funktion am Rest R¹ ist prinzipiell verantwortlich für die Zellgängigkeit der erfindungsgemäßen Verbindungen. Überraschenderweise bleibt die Zellgängigkeit der erfindungsgemäßen Verbindungen erhalten bzw. wird nur geringfügig schlechter, wenn nicht an jeder möglichen Position ein substituierter Rest R¹ vorhanden ist, also die Anzahl der Phosphonsäureester- bzw. Phosphonsäure-Funktionen und damit die Anzahl der asymmetrischen Zentren in den erfindungsgemäßen Verbindungen reduziert ist.

In diesem Zusammenhang bleibt auch die gute Zellgängigkeit der erfindungsgemäßen Verbindungen in lebendem Gewebe erhalten. So wurden in einem Experiment Medaka-Fische zwei Tage lang in einer Lösung von erfindungsgemäßen Verbindungen, welche mit einem Fluoreszenz-Farbstoff markiert waren, gehalten. Dann wurden die Fische in frisches Wasser umgesetzt, um die erfindungsgemäßen Verbindungen, die nicht in das Gewebe Magen-Darm-Trakt eingedrungen sind, aus dem Magen-Darm-Trakt wieder heraus zu waschen. Anschließend wurden die Fische an verschiedenen Tagen unter einem Fluoreszenz-Mikroskop untersucht. Die Ergebnisse zeigen, dass sich die erfindungsgemäßen Verbindungen sowohl im Magen-Darm-Trakt als auch in der Schwimmblase des Medaka-Fisches anlagern. Das Eindringen in die Darmwand des Fisches konnte auch durch Gewebeschnitte des Darms nachgewiesen werden. Dies macht die erfindungsgemäßen Verbindungen besonders wertvoll für die Bekämpfung von Krankheiten des Magen-Darm-Trakts, wie beispielsweise Darmkrebs, Morbus Crohn oder für die Behandlung von Fettleibigkeit.

Die Anzahl und die Reihenfolge der Reste R¹, die mit einer Phosphonsäureester- bzw. Phosphonsäure-Funktion substituiert sind, kann erfindungsgemäß frei gewählt werden. So kann beispielsweise jeder, jeder zweite, jeder dritte, jeder vierte, jeder fünfte, jeder sechste, jeder siebte, jeder achte, jeder neunte oder jeder zehnte Rest R¹ mit einer Phosphonsäureester- bzw. Phosphonsäure-Funktion substituiert sein. Die Substitutionen mit den Phosphonsäureester- bzw. Phosphonsäure-Funktionen können regelmäßig sein oder an beliebigen Positionen vorliegen.

Ferner können auch mehrere Reste R¹ aufeinander folgend mit einer Phosphonsäureester- bzw. Phosphonsäure-Funktion substituiert sein (benachbarte Anordnung). Dabei können in der Verbindung der allgemeinen Formel I auch mehrere dieser benachbarten Anordnungen enthalten sein.

Es können aber auch beispielsweise nur einzelne Reste R¹ an beliebigen Positionen mit einer Phosphonsäureester- bzw. die Phosphonsäure-Funktion substituiert sein.

Die Positionen mit den einzelnen, aufeinander folgenden Reste R¹, die mit einer Phosphonsäureester- bzw. die Phosphonsäure-Funktion substituiert sind, können beliebig sein.

Bei (den erfindungsgemäßen Verbindungen haben die Erfinder ein neues Wirkprinzip bei weiterhin guter Zellgängigkeit sowie eine überraschend starke Wirkung festgestellt.

Bei in-vitro-Zellexperimenten mit den erfindungsgemäßen Verbindungen konnten bei der Erst-Infektion von Wirtszellen (erstes Experiment) durch HI-Viren in der ersten Viren-Tochtergeneration eine scheinbar nur geringe Reduktion der Anzahl der gebildeten Viren gegenüber einer unbehandelten Kontrolle beobachtet werden. Dies würde auf einen schwachen Antisense-Effekt, wie es der Fachmann bislang versteht, hindeuten.

Die Anzahl der HI-Viren wurde dabei über einen standardisierten quantitativen p24-ELISA-Assay ermittelt, da die Menge des gebildeten viralen Proteins p24 Menge generell als proportional zur Anzahl der gebildeten HI-Viren angesehen wird.

Das infektiöse Zellmedium (Überstand) der ersten Viren-Tochtergeneration kann durch Abzentrifugieren von den Wirtszellen isoliert werden. Mit diesem Überstand können in Folge-Experimenten neue, zuvor nicht infizierte Wirtszellen infiziert werden (Zweit-Infektion), wobei keine neuerliche Zugabe der erfindungsgemäßen Verbindungen erfolgt. Um innerhalb des Messbereiches des p24-Assays zu bleiben, wird dieser Überstand zuvor verdünnt (beispielsweise 1/5000).

In einem Folge-Experiment werden die verdünnten Überstände, sowohl diejenigen der mit den erfindungsgemäßen Verbindungen behandelten Wirtszellen als auch diejenigen der unbehandelten Kontrollen, jeweils zu zuvor nicht infizierten Wirtszellen gegeben. Dabei erfolgt keine neuerliche Zugabe der erfindungsgemäßen Verbindungen. Jetzt resultiert eine starke Reduktion der gemessenen p24-Menge in der zweiten Tochtergeneration bei den im ersten Experiment behandelten Wirtszellen. Im Gegensatz dazu bewirkt der Überstand der unbehandelten Kontrolle eine starke Erhöhung der p24-Menge in der zweiten Tochtergeneration.

Dieses überraschende Ergebnis steht in Widerspruch zu den Ergebnissen aus der ersten Tochtergeneration. Während bei dem ersten Experiment trotz des Vorhandenseins von Wirkstoff, eine scheinbar nur geringe Wirkung erzielt wird, ist bei dem Folge-Experiment bei weitgehender Abwesenheit von Wirkstoff eine starke Wirkung zu sehen.

Dieser Widerspruch ist nur durch den neuen Wirkmechanismus der erfindungsgemäßen Verbindungen auflösbar.

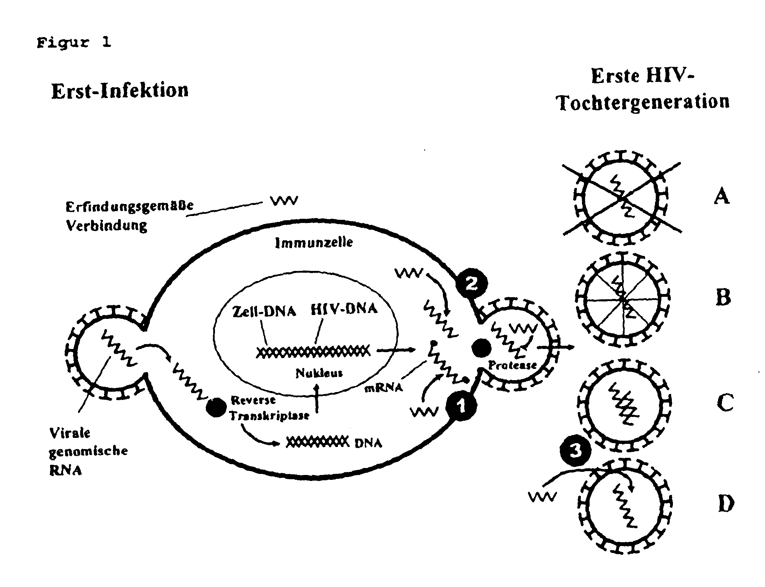

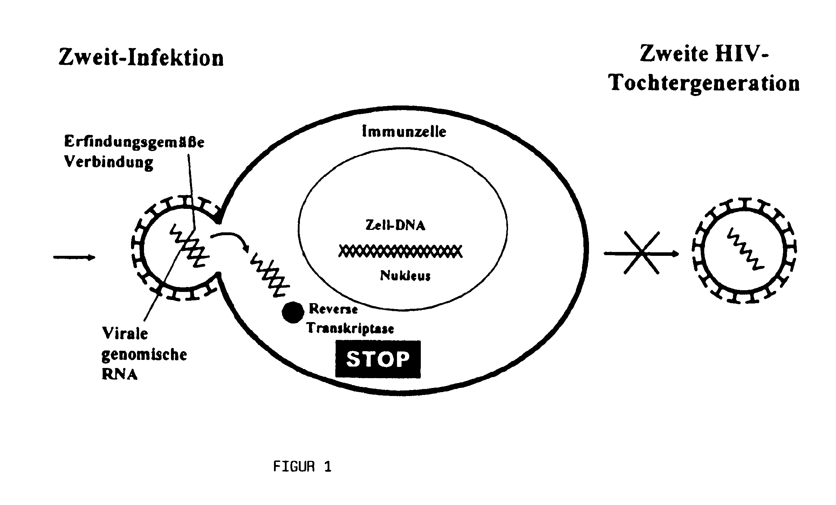

Figur 1 zeigt schematisch den neuen Wirkmechanismus.

Bei der Erstinfektion einer Wirtszelle durch einen HI-Virus entlässt dieser seine virale genomische RNA in das Cytosol. Anschließend wird diese durch virale Reverse Transkriptase in DNA umgeschrieben und in das Genom der Wirtszelle integriert. Bei Aktivierung der Wirtszelle wird einerseits virale genomische RNA gebildet, andererseits kommt es zur Bildung von viraler mRNA, welche durch Translation in virale Proteine übersetzt werden kann.

Werden die Wirtszellen mit den erfindungsgemäßen Verbindungen behandelt, so sind diese in der Lage, ohne weitere Hilfsmittel (z. B. Transfektionsreagentien) in die Zelle einzudringen. Bei Vorhandensein viraler mRNA mit komplementärer Sequenzfolge können die erfindungsgemäßen Verbindungen an diese anlagern. Dadurch wird die Translation in bestimmte virale Proteine blockiert. Dies ist ein Antisense-Effekt (1). Dadurch wird z.B. die Bildung neuer Viren unterbunden (Fall A). Durch das Fehlen bestimmter viraler Proteine kann z.B. auch das Ausreifen eines Viruspartikels nach dem Ausknospen aus der Wirtszelle behindert werden. In diesem Fall entstehen nicht infektiöse Viren (Fall B). Im p24-Assay kann allerdings nicht zwischen solchen, nicht infektiösen Viren, und solchen, die weiterhin infektiös sind, unterschieden werden. Ein an sich starker Antisense-Effekt wird auf diese Weise falsch als schwacher Antisense-Effekt detektiert.

Außer an die mRNA (klassischer Antisense-Effekt) können sich die erfindungsgemäßen Verbindungen überraschenderweise auch gleichzeitig an die genomische RNA mit komplementärer Sequenzfolge (antigenomischer Effekt) des HI-Virus (RNA⁺-Virus) anlagern (2). Dies kann innerhalb der Wirtszelle und/oder beim Ausknospen geschehen.

Da die Zellmembran des Virus zum größten Teil aus der Membran der Wirtszelle besteht, können die erfindungsgemäßen Verbindungen allerdings auch nach dem Ausknospen der Viren deren Membranen durchdringen und dann an die komplementäre Sequenzfolge der viralen genomischen RNA (3) anlagern.

Diese Viren (C und D) sind noch in der Lage, weitere Wirtszellen zu infizieren. Die, an die virale genomische RNA angelagerten erfindungsgemäßen Verbindungen wirken allerdings als Blockade für die virale Reverse Transkriptase. Damit ist die Transkription der RNA in DNA nicht mehr möglich und der Replikationszyklus der Viren an dieser Stelle unterbrochen.

Die erfindungsgemäßen Verbindungen können sich demnach gleichzeitig sowohl an komplementäre virale genomische RNA als auch an komplementäre virale mRNA anlagern. Bei Anlagerung an genomische RNA können die erfindungsgemäßen Verbindungen die erste Wirtszelle wieder verlassen und in eine zweite, zuvor nicht infizierte Wirtszelle eindringen. Dies führt zu einer überraschenden, über zwei Virengenerationen beobachtbaren Wirksamkeit.

Durch die Anlagerung an genomische RNA können dadurch auch latente Viren bekämpft werden. Beispielsweise können die derzeit auf dem Markt befindlichen HIV-Medikamente nur replizierende HI-Viren bekämpfen. Mit einer Kombinationstherapie aus Verbindungen, die an genomische RNA anlagern (wie beispielsweise die erfindungsgemäßen Verbindungen), und Verbindungen, welche die Virenlast durch Blockade der Virenreplikation niedrig halten, können erfindungsgemäß sowohl replizierende Viren als auch latente Viren gleichzeitig bekämpft werden und somit eine Ausrottung der entsprechenden Viren im menschlichen Organismus erreicht werden.

Die erfindungsgemäßen Verbindungen zeigen einen überlegenen Antisense-Effekt und eine stärkere Wirksamkeit gegenüber den in EP 1157031 beschriebenen stereochemisch uneinheitlichen Verbindungen. Bei beiden Verbindungsklassen steigt die Stärke des Antisense-Effekts mit zunehmender Anzahl der Monomere, die zur Herstellung der Oligomere verwendet wurden, d.h. mit größeren Werten von n. Selbst deutlich kürzere erfindungsgemäße Verbindungen sind jedoch überraschenderweise längeren, stereochemisch uneinheitlichen Oligomeren gemäß EP 1157031 in der Wirkung deutlich überlegen. Dies macht die erfindungsgemäßen Verbindungen auch für weitere Anwendungen besonders wertvoll.

Erfindungsgemäße Verbindungen sind vor allem durch ihre Fähigkeit, an komplementäre Nukleinsäuresequenzen zu binden, zur Behandlung von unterschiedlichen Krankheiten von großem Interesse. Mit diesen Verbindungen können Krankheiten bekämpft werden, die auf das Vorhandensein nichtkörpereigener DNA oder RNA zurückzuführen sind. Als Beispiele sind hier vor allem Krankheiten, verursacht durch Viren wie beispielsweise HIV, Hepatitis B und Hepatitis C oder HPV, zu erwähnen.

Aber auch Krankheiten, die auf eine Überexpression von körpereigener mRNA zurückzuführen sind, können mit den erfindungsgemäßen Verbindungen bekämpft werden. Als Beispiele sind hierbei die verschiedensten Krebsarten wie beispielsweise Hautkrebs, Lungenkrebs, Leberkrebs, Prostatakrebs, Leukämie oder Gehirntumore.

Entsprechende Experimente mit erfindungsgemäßen Verbindungen zeigten eine Reduzierung der Zellproliferation in der Her2/neu überexprimierenden Brustkrebszelllinie MDA453 um 33 %. In diesem Experiment wurden die Zellinien in Zellkultur vier Tage mit einer erfindungsgemäßen Verbindung, die eine komplementäre Match-Sequenz zur Her2/neu mRNA darstellt, inkubiert. Eine negativ Kontrolle, die keine komplementäre Match-Sequenz zur Her2/neu mRNA darstellt, zeigte keine Reduzierung der Zellproliferation.

Die Verringerung der Konzentration von Her2/neu in Mamma-Karzinomen erhöht die Überlebensrate in Kombination mit konventionellen Chemotherapeutika bei Brustkrebspatienten deutlich. (Piccart-Gebhart MJ et al.: Herceptin® Adjuvant (HERA) Trial Study Team. Trastuzumab after adjuvant chemotherapy in HER2-positive breast cancer. N Engl J Med. 2005 Oct 20;353(16):1659-72)

Weiterhin können Entzündungskrankheiten wie beispielsweise Asthma oder Schuppenflechte, neurologische Krankheiten wie beispielsweise Parkinson oder metabolische Krankheiten wie erhöhte Cholesterin-Werte mit den erfindungsgemäßen Verbindungen bekämpft werden.

Entsprechende Experimente mit erfindungsgemäßen Verbindungen, die gegen die Expression des Cholesterin-Träger-Proteins ApoB100 gerichtet waren, zeigte in Mäusen ein Reduktion des Gehalts vom ApoB100 um 41% im Vergleich zu der nur mit PBS-Puffer behandelten Kontrollgruppe. Gleichzeitig wurde eine Reduktion von ApoB48, einem weiteren Cholesterin-Träger-Protein, das für den Transport von Cholesterin vom Dünndarm zur Leber verantwortlich ist, um 32% beobachtet. Die Reduktion von ApoB100 und ApoB48 resultierte in einer Gesamt-Reduktion des Cholesterinspiegels um 25%. In diesem Experiment wurde Mäusen an drei aufeinander folgenden Tagen einmal täglich erfindungsgemäße Verbindungen in einer Konzentration von 25 mg/kg intravenös verabreicht und anschließend am vierten Tag das Mäuseblut analysiert.

Eine Reduktion von ApoB100 und ApoB48 ist wertvoll im Hinblick auf die Bekämpfung von Krankheiten, die mit Arteriosklerose und erhöhten Cholesterin-Werten, insbesondere im Hinblick auf Hoch-Risiko-Gruppen, zusammenhängen.

Auch die Verwendung dieser Wirkstoffe zur Herstellung von Arzneimitteln zur Vorbeugung und/oder Behandlung von Erkrankungen, ist Gegenstand der vorliegenden Erfindung. Im Allgemeinen werden erfindungsgemäße Verbindungen unter Anwendung der bekannten und akzeptablen Modi, entweder einzeln oder in Kombination mit einem beliebigen anderen therapeutischen Mittel verabreicht. Die Verabreichung kann z.B. auf einem der folgenden Wege erfolgen: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachs, Fett, Polyole einsetzen. Zur Herstellung von flüssigen Lösungen und Sirups kann man Arzneimittelträgerstoffe wie z.B. Wasser, Alkohole, wässrige Salzlösung, wässrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachs, Fett und Polyole verwenden. Für Aerosol-Formulierungen kann man komprimierte Gase, die für diesen Zweck geeignet sind, wie z.B. Sauerstoff, Stickstoff und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Die erfindungsgemäßen Verbindungen lassen sich beispielsweise mittels in der Literatur beschriebenen Verfahren durch Umsetzung von Verbindungen der allgemeinen Formel **II** in an sich bekannter Weise (z.B. L. Christensen, R. Fitzpatrick, B. Gildea, K.H. Petersen, H.F. Hansen, T. Koch, M. Egholm, O. Buchardt, P.E. Nielsen, J. Coull, R.H. Berg, J.Pept.Sci. 3, 1995, 175-183. T. Koch, H.F. Hansen, P. Andersen, T. Larsen, H.G. Batz, K. Otteson, H. Örum, J.Pept.Res. 49, 1997, 80-88. F. Bergmann, W. Bannwarth, S. Tam, Tetrahedron Lett. 36, 1995, 6823-6826) herstellen.

In den Verbindungen der allgemeinen Formel **II** 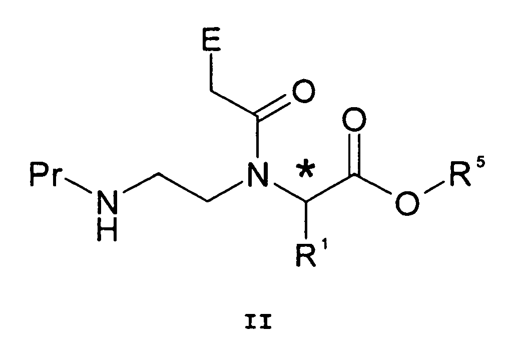 ist der Rest R⁵ z.B. ein H-Atom oder ein Allyl-, Benzyl-, Ethyl-, Methyl-Rest oder ein lösliches oder unlösliches Polymer.

Pr ist ein H-Atom oder eine abspaltbare Aminschutzgruppe. Die Aminschutzgruppe muß in Gegenwart der Nukleobasen-Schutzgruppen selektiv abspaltbar sein. Vorzugsweise ist Pr ein H-Atom, eine Oxocarbamat- oder eine Thiocarbamat-Schutzgruppe, am stärksten bevorzugt ist Pr ein H-Atom oder eine Fmoc-, Boc-, Cbz-, Mmt- oder eine Bhoc-Schutzgruppe.

E und der Rest R¹ sind wie oben definiert.

Das asymmetrische Zentrum (*), an das der Rest R¹ bindet, kann die R- oder S-Konfiguration aufweisen.

Die Verbindungen der allgemeinen Formel **II** können zum Beispiel nach folgendem Verfahren hergestellt werden.

Herstellung der Verbindungen der allgemeinen Formel **II** mit R-Konfiguration am asymmetrischen Zentrum:

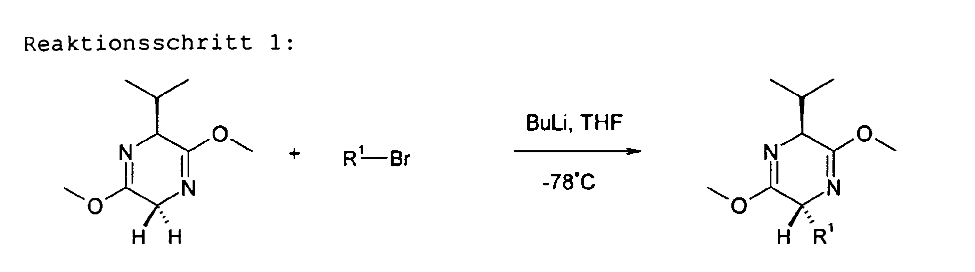

Ausgehend von der S-Konfiguration des Pyrazin-Edukts kann die Durchführung beispielsweise wie in der Literatur beschrieben (U. Schöllkopf, U. Busse, R. Lonsky, R. Hinrichs, Liebigs Ann. Chem. 1986, 2150 -2163; A. Schick, T. Kolter, A. Giannis, K. Sandhoff, Tetrahedron 51, 1995, 11207-11218) erfolgen.

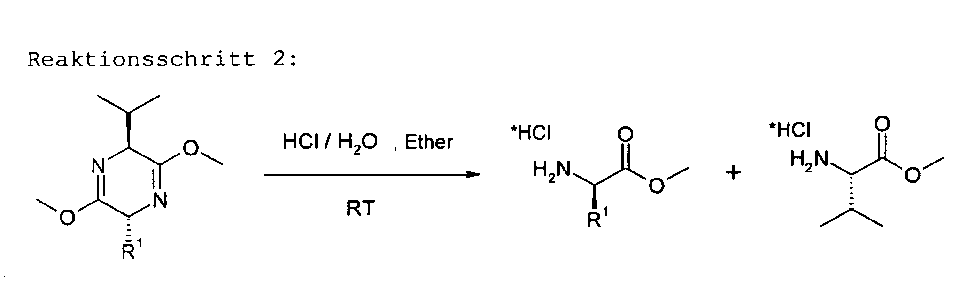

Die Durchführung kann beispielsweise wie in der Literatur beschrieben (U. Schöllkopf, U. Busse, R. Lonsky, R. Hinrichs, Liebigs Ann. Chem. 1986, 2150 -2163) erfolgen.

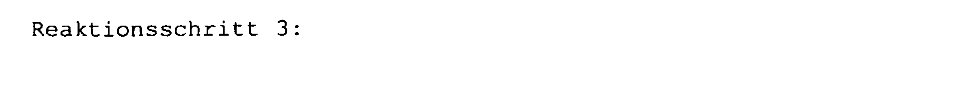

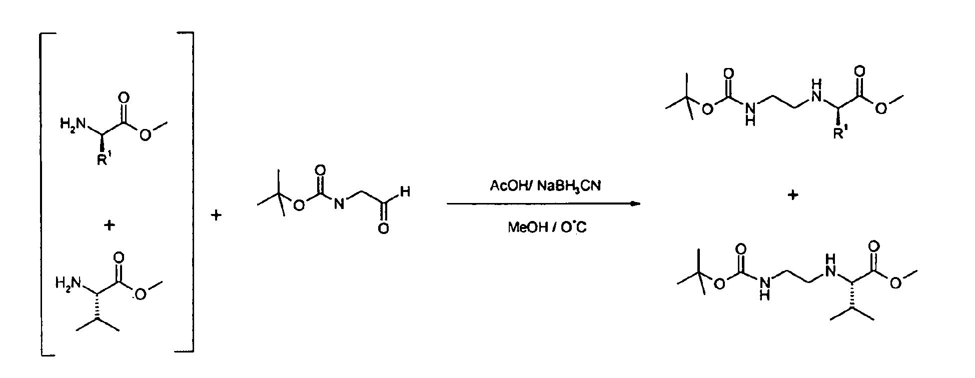

Das Produktgemisch aus Reaktionsschritt 2 kann, nach Freisetzung der Amine aus ihren Hydrochloriden durch eine Base (z.B. NaHCO₃, NH₃), in die Folgereaktion eingesetzt werden. Diese, eine reduktive Aminierung, kann wie in der Literatur beschrieben (G. Haaima, A. Lohse, O. Buchardt, P.E. Nielsen, Angew. Chem. Int. Ed. Engl. 35, 1996, No 17, 1939-1942) durchgeführt werden.

Statt Natriumcyanoborhydrid können auch andere Reduktionsmittel wie z. B. Wasserstoff und ein Katalysator (z. B. Pd/C) verwendet werden. Die Reaktionsprodukte werden chromatographisch aufgetrennt.

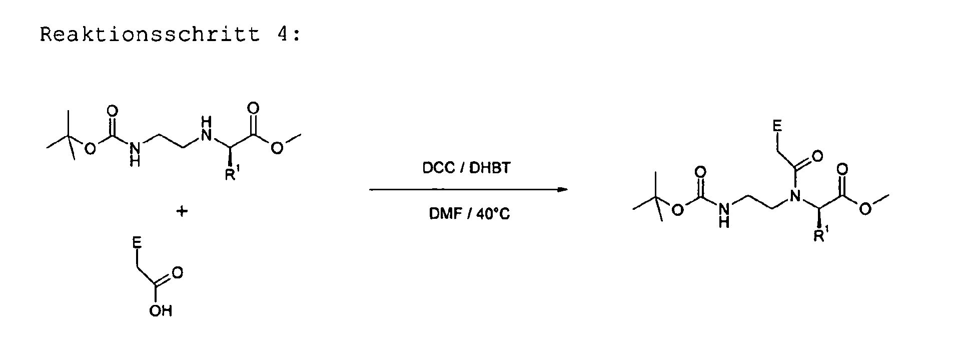

Die Durchführung kann beispielsweise wie in der Literatur beschrieben (G. Haaima, A. Lohse, O. Buchardt, P.E. Nielsen, Angew. Chem. Int. Ed. Engl. 35, 1996, No 17, 1939-1942) erfolgen. Dabei können auch andere Kopplungsreagentien an Stelle von DCC /DHBT eingesetzt werden.

Die Herstellung der Verbindung E-CH₂-COOH (zum Beispiel C(PG)-CH₂-COOH, A(PG)-CH₂-COOH, G(PG)-CH₂-COOH oder T-CH₂-COOH, J(PG)-CH₂-COOH wobei A = Adeninyl, C = Cytosinyl, G = Guaninyl, T = Thyminyl, J = Pseudoisocytosinyl PG = Schutzgruppe wie beispielsweise Benzyloxycarbonyl (Z), Benzyl (Bzl), Acetyl(Ac) oder Anisoyl (An)) kann beispielsweise wie in der Literatur beschrieben (S.A. Thomson, J.A. Josey, R.Cadilla, M.D. Gaul, F.C. Hassmann, M.J. Lazzio, A.J. Pipe, K.L. Reed, D.J. Ricca, R.W. Wiether, S.A. Noble, Tetrahedron 51, 1995, 6179-6194) erfolgen.

Weitere mögliche Schutzgruppen sind ebenfalls in der Literatur beschrieben (G. Breipohl, D.W. Will, A. Peymann, E. Uhlmann, Tetrahedron 53, 1997, 14671-14686; T. Kofoed, H.F. Hansen, H. Orum, T. Koch, J. Peptide Sci., 7, 2001, 402-412)

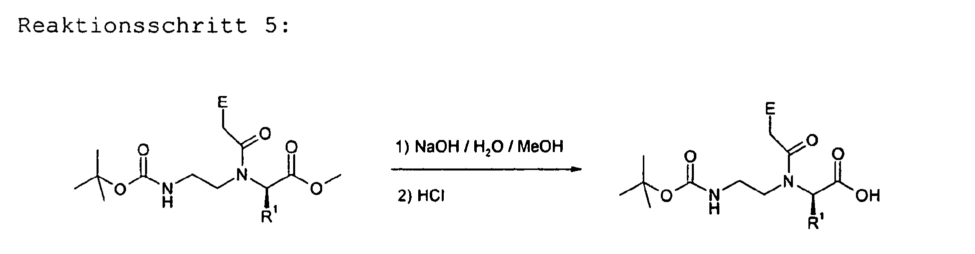

Die Durchführung kann beispielsweise wie in der Literatur beschrieben (G. Haaima, A. Lohse, O. Buchardt, P.E. Nielsen, Angew. Chem. Int. Ed. Engl. 35, 1996, No 17, 1939-1942) erfolgen.

Zur einfacheren Beschreibung der Verbindungen der allgemeinen Formel **II,** die als Produkte in dem Reaktionsschritt 5 entstehen, werden folgende Abkürzungen verwendet:
Wird beispielsweise A(PG)-CH₂-COOH im Reaktionsschritt 4 eingesetzt, so erhält man die entsprechende Verbindung der allgemeinen Formel **II,** die ein asymmetrisches Zentrum aufweist. Diese Verbindung wird hier im Allgemeinen als A^{R}(PG) abgekürzt. Dabei steht A für die Nukleobase in der Verbindung der allgemeinen Formel **II** mit einem asymmetrischen Zentrum, das hochgestellte R steht für die R-Konfiguration der Verbindung und das PG steht für die Schutzgruppe an der Nukleobase.
Wird beispielsweise Phenylessigsäure im Reaktionsschritt 4 eingesetzt so erhält man eine Verbindung der allgemeinen Formel **II** mit einem asymmetrischen Zentrum, die als P^{R} abgekürzt wird.

Die entsprechenden Verbindungen der allgemeine Formel **II** ohne asymmetrisches Zentrum (R¹ = H) werden analog zu den Verbindungen der allgemeinen Formel **II** mit einem asymmetrischen Zentrum abgekürzt mit dem Unterschied, das anstatt dem Großbuchstaben für die Nukleobase und dem hochgestellten Buchstaben für die Konfiguration (z.B. A^{R}) der entsprechende Kleinbuchstabe a verwendet wird. Beispielsweise wird eine Verbindung der allgemeinen Formel **II** ohne asymmetrisches Zentrum mit PG-geschütztem C als Nukleobase als c(PG) abgekürzt.

Für die Herstellung der Verbindungen der allgemeinen Formel **II** mit S-Konfiguration am asymmetrischen Zentrum wird im Reaktionsschritt 1 das Pyrazin-Edukt mit der R-Konfiguration eingesetzt und die Reaktionsschritte 1 bis 5 analog durchgeführt. Man erhält dann beispielsweise eine Verbindung der allgemeinen Formel **II** mit der Abkürzung A^{S}(PG).

Die erfindungsgemäßen Verbindungen lassen sich beispielsweise mittels Festphasensynthese durch Umsetzung von Verbindungen der allgemeinen Formel **II** in an sich bekannter Weise herstellen. Nach der Festphasensynthese werden die Schutzgruppen an den Nukleobasen abgespalten, so dass man Verbindungen der allgemeinen Formel **I** erhält, die wie folgt abgekürzt werden:

Beispielsweise wird eine erfindungsgemäße Verbindung, die nur aus Verbindungen der allgemeinen Formel **II** mit einem asymmetrischen Zentrum mit R-Konfiguration hergestellt und welche im letzten Schritt Acetyl-gecappt und danach als primäres Amid vom Harz abgespalten wurde, abgekürzt als Ac-A^{R}G^{R}T^{R}C^{R}G^{R}T^{R}T^{R}T^{R}C^{R}A^{R}A^{R}C^{R}C^{R}- NH₂.

Beispielsweise wird eine erfindungsgemäße Verbindung, die aus Verbindungen der allgemeinen Formel **II** mit einem asymmetrischen Zentrum mit R-Konfiguration und Verbindungen der allgemeinen Formel **II** ohne asymmetrisches Zentrum hergestellt und welche im letzten Schritt mit Fluorescein gelabelt und danach als primäres Amid vom Harz abgespalten wurde, abgekürzt als Flu-A^{R}gT^{R}C^{R}G^{R}tT^{R}T^{R}C^{R}aaC^{R}c-NH₂.

Beispielsweise wird eine erfindungsgemäße Verbindung, die an einem Boc-Gly-PAM-MBHA-Harz nur aus Verbindungen der allgemeinen Formel **II** mit einem asymmetrischen Zentrum mit S-Konfiguration sowie einer L-Aminosäure wie Boc-ε-(L)-Trimethyllysin-iodid (Boc-ε(L)TML-iodid) hergestellt und welche im letzten Schritt Acetyl-gecappt und danach als primäres Amid vom Harz abgespalten wurde, abgekürzt als Ac-ε(L)TML-A^{S}G^{S}T^{S}C^{S}G^{S}T^{S}T^{S}T^{S}C^{S}A^{S}A^{S}C^{S}C^{S}-Gly-NH₂.

Beispielsweise wird eine erfindungsgemäße Verbindung, die an einem Boc-Gly-PAM-MBHA-Harz nur aus Verbindungen der allgemeinen Formel **II** mit einem asymmetrischen Zentrum mit S-Konfiguration sowie vier L-Aminosäuren wie Boc-ε-(L)-Trimethyllysin-iodid (Boc-ε(L)TML-iodid) hergestellt und welche im letzten Schritt Acetyl-gecappt und danach als primäres Amid vom Harz abgespalten wurde, abgekürzt als Ac-ε(L)TML-ε(L)TML-ε (L) TML- ε (L) TML-A^{S}G^{S}T^{S}C^{S}G^{S}T^{S}T^{S}T^{S}C^{S}A^{S}A^{S}C^{S}C^{S}-Gly-NH₂.

Beispielsweise wird eine erfindungsgemäße Verbindung, die an einem Boc-Gly-PAM-MBHA-Harz aus Verbindungen der allgemeinen Formel **II** mit einem asymmetrischen Zentrum mit R-Konfiguration und Verbindungen der allgemeinen Formel II ohne asymmetrisches Zentrum, Glycin sowie zwei Aminosäuren wie 4-(Diethoxy-phosphoryl)-2-(tert.butoxycarbonylamino)-buttersäure (Boc-DEPABS) hergestellt und welche im letzten Schritt Acetyl-gecappt und danach als primäres Amid vom Harz abgespalten wurde, abgekürzt als Ac-(DEPABS)₂-Gly-gcgtG^{R}tG^{R}ggaagG^{R}cA^{R}g-Gly-NH₂.

Beispielsweise wird eine erfindungsgemäße Verbindung, die an einem Boc-Gly-PAM-MBHA-Harz aus Verbindungen der allgemeinen Formel **II** mit einem asymmetrischen Zentrum mit R-Konfiguration und Verbindungen der allgemeinen Formel **II** ohne asymmetrisches Zentrum, den Aminosäuren L-Lysin (L-Lys), L-Arginin (L-Arg) L-Valin (L-Val) hergestellt und welche im letzten Schritt Acetyl-gecappt und danach als primäres Amid vom Harz abgespalten wurde, abgekürzt als Ac-(L-Lys)-(L-Lys)-(L-Lys)-(L-Arg) - (L-Lys) - (L-Val) -agCtC^{R}cT^{R}CgcccT^{R}tG^{R}C-Gly-NH₂.

Beispielsweise wird eine erfindungsgemäße Verbindung, die an einem Boc-Gly-PAM-MBHA-Harz aus Verbindungen der allgemeinen Formel **II** mit einem asymmetrischen Zentrum mit R-Konfiguration und Verbindungen der allgemeinen Formel **II** ohne asymmetrisches Zentrum, der Aminosäuren Boc-ε-(L)-Trimethyllysin-iodid (Boc-ε(L)TML-iodid) und dem Chelator 1,4,7,10-Tetraazacyclododecane-1,4,7,10-tetraessigsäure-tri-tert.butylester (DOTA) hergestellt danach als primäres Amid vom Harz abgespalten wurde, abgekürzt als DOTA-ε(L)TML-C^{R}aG^{R}tT^{R}aG^{R}gG^{R}tT^{R}aG^{R}-Gly-NH₂.

### Beispiele

### Beispiel 1: Herstellung von (2R,5S)-2-(2-(Diethoxy-phosphoryl)ethyl)-2,5-dihydro-3,6-dimethoxy-5-isopropyl-pyrazin

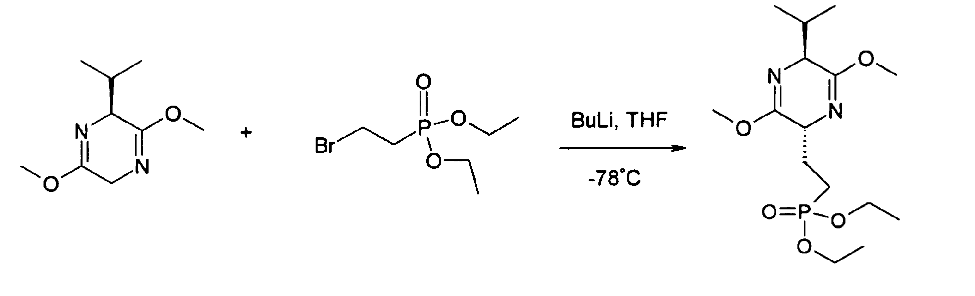

0,52 mol (S)-2,5-Dihydro-3,6-dimethoxy-2-isopropylpyrazin werden in 400 ml absolutem THF unter Argon gelöst und auf - 78°C gekühlt. Unter Rühren werden 200 ml einer 2,7 M Butyllithium-Lösung (in Heptan) (0,54 mol) langsam zugetropft. Anschließend tropft man eine Lösung von 0,52 mol Diethyl-(2-bromethyl)-phosphonat in 300 ml absolutiertem THF langsam unter Rühren zu und rührt weitere 3 h bei -78°C. Dann werden 11,7 ml (ca. 0,2 mol) wasserfreie Essigsäure langsam zugegeben. Man lässt die Reaktionsmischung dann langsam auf Raumtemperatur erwärmen. Das Lösemittel wird entfernt, der Rückstand in 600 ml Diethylether aufgenommen und mit 200 ml Wasser gewaschen. Die wässrige Phase wird noch 3-mal mit je 100 ml Diethylether extrahiert. Die vereinigten Etherphasen werden über MgSO₄ getrocknet, filtriert und das Lösemittel wird im Vakuum entfernt. Der Rückstand wird in Et₂O/Hexan (1/10) aufgenommen und über ein Kieselgelbett filtriert. Dabei wird erst mit Et₂O /Hexan (1/5) eluiert.
Ausbeute: ca. 70 %, Gelbe Flüssigkeit
**¹H-NMR**(CDCl₃): 0.71, 1.04 (d, 6H, CH (C*H*₃) ₂), 1.33 (t, 6H, P(O)(OCH₂C*H*₃) ₂), 1.68-2.25 (m, 4H, CHC*H*₂C*H*₂P), 3.65, 3.67 (s, 6H, OC*H*₃), 4.02 (m, 1H), 4.10-4.20 (m, 4H, P(O)(OC*H*₂CH₃)₂).

### Beispiel 2: Herstellung von (2R,5S)-2-(8-(Dibenzyloxy-phosphoryl)octyl)-2,5-dihydro-3,6-dimethoxy-5-isopropyl-pyrazin

Analog dem Herstellungsverfahren in Beispiel 1 wird ausgehend von (S)-2,5-Dihydro-3,6-dimethoxy-2-isopropylpyrazin und Dibenzyl-(8-bromoctyl)-phosphonat (2R,5S)-2-(8-(Dibenzyloxy-phosphoryl)octyl)-2,5-dihydro-3,6-dimethoxy-5-isopropyl-pyrazin hergestellt.

### Beispiel 3: Herstellung von (2S,5R)-2-(4-(Dicyclohexyloxy-phosphoryl)but-2-enyl)-2,5-dihydro-3,6-dimethoxy-5-isopropyl-pyrazin

Analog dem Herstellungsverfahren in Beispiel 1 wird ausgehend von (R)-2,5-Dihydro-3,6-dimethoxy-2-isopropylpyrazin und Dicyclohexyl-(4-brom-but-2-enyl)-phosphonat (2S,5R)-2-(4-(Dicyclohexyloxy-phosphoryl)but-2-enyl)-2,5-dihydro-3,6-dimethoxy-5-isopropyl-pyrazin hergestellt.

### Beispiel 4: Herstellung von (2R)-2-[2-(tert.Butoxycarbonylamino)ethyl]-amino-4-(diethoxy-phosphoryl)-buttersäuremethylester

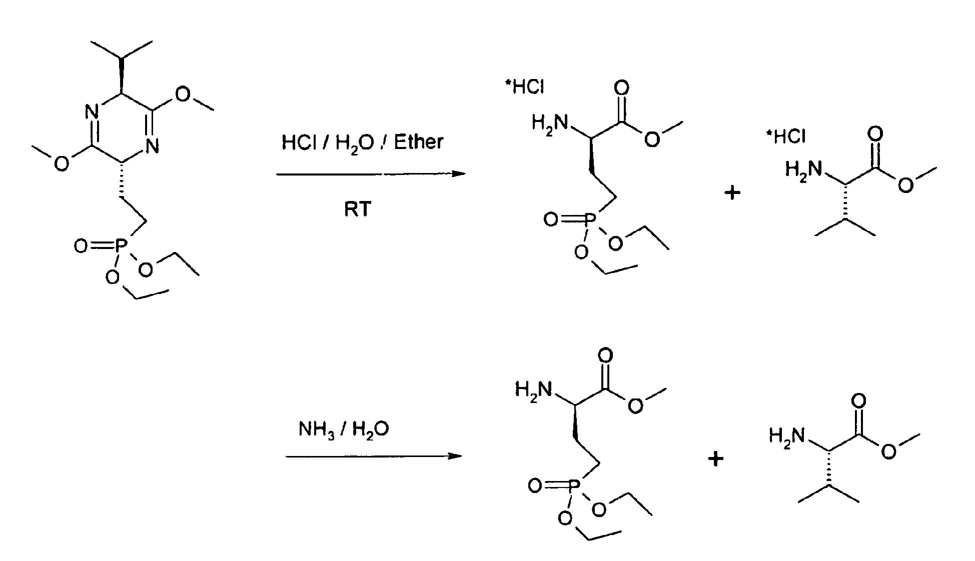

0,38 mol (2R,5S)-2-(2-(Diethoxy-phosphoryl)ethyl)-2,5-dihydro-3,6-dimethoxy-5-isopropyl-pyrazin werden in 400 ml Diethylether gelöst. Zu dieser Lösung werden 1150 ml einer 1N wässriger HCl-Lösung zugefügt. Nach 60 min ist die Reaktion beendet und der Ether wird entfernt. Soll das Produkt gelagert werden, wird auch das Wasser komplett im Vakuum entfernt. Soll das Produkt gleich weiter umgesetzt werden, wird das Wasser etwa zur Hälfte abrotiert, dann wird der pH-Wert des Reaktionsgemisches mit Ammoniaklösung auf 8-9 eingestellt. Die basische Lösung wird 6x mit Dichlormethan extrahiert, wobei jedes Mal der pH-Wert kontrolliert und nötigenfalls korrigiert wird. Die Dichlormethan-Phasen werden vereinigt, mit MgSO₄ getrocknet, und das Lösungsmittel im Vakuum entfernt. Das resultierende gelbe Öl wird sofort in der Folgereaktion, der reduktiven Aminierung eingesetzt.

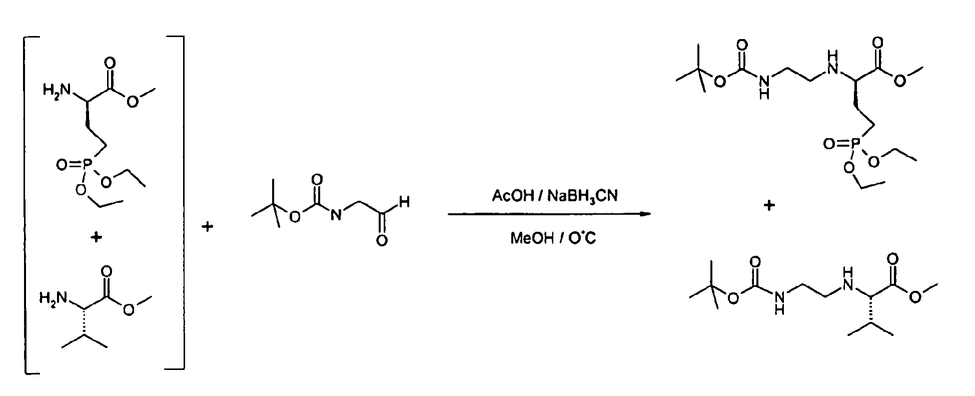

Das gelbe Öl (angenommen wird ein vollständiger Umsatz) wird in 600 ml Methanol aufgenommen und auf 0°C gekühlt. Anschließend werden 0,76 mol N-Boc-Aminoacetaldehyd zugegeben. Nachdem 30 min. bei 0°C gerührt wurde, werden erst 0,90 mol wasserfreie Essigsäure, dann 0,40 mol Natriumcyanoborhydrid zugefügt. Die Reaktionsmischung wird bei 0°C gerührt, bis die Gasentwicklung beendet ist, dann wird das Lösemittel am Rotationsverdampfer entfernt. Der Rückstand wird in Essigsäureethylester aufgenommen (ca. 600 ml) und einmal mit gesättigter NaHCO0₃-Lsg. (ca. 200 ml) und einmal mit gesättigter NaCl-Lsg. (ca. 100 ml) gewaschen. Die organische Phase wird mit M₉SO₄ getrocknet und filtriert. Anschließend wird das Lösungsmittel im Vakuum entfernt.

Die weitere Aufreinigung erfolgt durch SPE über eine mit Kieselgel gefüllte Glasfritte. Verunreinigungen und unerwünschte Produkte werden zuerst mit Hexan/Essigester 1:1, dann mit reinem Essigester eluiert. Das gewünschte Produkt wird schließlich durch Extraktion mit 10% Methanol in Dichlormethan erhalten.

Nach Entfernen des Lösemittels werden ca. 75%, Produkt als gelbes viskoses Öl erhalten.
**¹H-NMR** (CDCl₃) : 1.35 (t, 6H, P(O)(OCH₂C*H*₃) ₂), 1.47 (s, 9H, C(C*H*₃)₃); 1.8-2.0 (m, 4H, CHC*H*₂CH₂P,), 2.5-2.6, 2.75-2.85, 3.0-3.4 (m, 4H, NC*H*₂C*H*₂N), 3.75 (s, 3H, OC*H*₃) , 4.0-4.2 (m, 4H, P (O) (OC*H*₂CH₃) ₂) .

### Beispiel 5: Herstellung von (2R)-2-[2-(tert.Butoxycarbonylamino)ethyl]-amino-10-(dibenzyloxy-phosphoryl)-decansäuremethylester

Analog dem Herstellungsverfahren in Beispiel 4 wird ausgehend von (2R,5S)-2-(8-(Dibenzyloxy-phosphoryl)octyl)-2,5-dihydro-3,6-dimethoxy-5-isopropyl-pyrazin (2R)-2-[2-(tert.Butoxycarbonylamino)ethyl]-amino-10-(dibenzyloxy-phosphoryl)-decansäuremethylester hergestellt.

### Beispiel 6: Herstellung von (2S)-2-[2-(tert.Butoxycarbonylamino)ethyl]-amino-6-(dicyclohexyloxy-phosphoryl)-hex-4-ensäuremethylester

Analog dem Herstellungsverfahren in Beispiel 4 wird ausgehend von (2S,5R)-2-(4-(Dicyclohexyloxy-phosphoryl)but-2-enyl)-2,5-dihydro-3,6-dimethoxy-5-isopropyl-pyrazin (2S)-2-[2-(tert.Butoxycarbonylamino)ethyl]-amino-6-(dicyclohexyloxy-phosphoryl)-hex-4-ensäuremethylester hergestellt.

### Beispiel 7: Herstellung von (R)-2-([2-{N4-Benzyloxycarbonylcytosin-1yl}-acetyl]-[2-tert.butoxycarbonylamino-ethyl]-amino)-4-(diethoxy-phosphoryl)-buttersäure-methylester

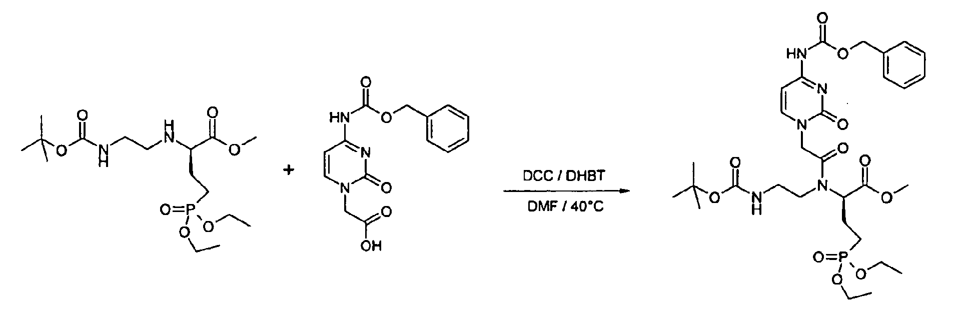

Zu einer gerührten Lösung aus 30,96 mmol 4-N-(Benzyloxycarbonyl)-cytosin-1-yl-essigsäure, und 30,96 mmol 3,4-Dihydro-3-hydroxy-4-oxo-1,2,3-benzotriazin (DHBT-OH) in 100 ml absolutem DMF werden 32,51 mmol DCC gegeben, und diese Lösung wird für 1 h bei 40°C gerührt. Anschließend werden 23,84 mmol (2R)-2-[2-(tert.Butoxycarbonylamino)ethyl]-amino-4-(diethoxy-phosphoryl)-buttersäuremethylester gegeben und bei 40° gerührt. Die Reaktion wird mit HPLC verfolgt und ist nach 3 Tagen beendet.

Vom Unlöslichen wird abfiltriert, das Lösemittel wird im Vakuum entfernt. Der Rückstand wird in Dichlormethan aufgenommen und über Nacht in den Kühlschrank gestellt. Dabei fällt weiterer Dicyclohexylharnstoff aus, der abfiltriert wird. Das Filtrat wird 2-3x mit verdünnter NaHCO₃-Lösung (1/3 ges. NaHCO₃-Lsg., 2/3 Wasser), 1-2x mit verdünnter KHSO₄-Lösung (1/3 gesättigter. KHSO₄-Lsg., 2/3 Wasser gewaschen, mit MgSO₄ getrocknet und einrotiert. Die weitere Aufreinigung erfolgt durch Lösen in Essigester und Stehen über Nacht im Kühlschrank, wonach eventuell weiterer ausgefallener Dicyclohexylharnstoff abfiltriert und das Lösemittel wieder entfernt wird. Das Rohprodukt wird dann in Dichlormethan gelöst (5 mL je 3 g Rohprodukt) und mit Diethylether (25 mL je 3 g Rohprodukt) und Hexan (5 mL je 3 g Rohprodukt) wieder ausgefällt. Das Lösemittel mit Verunreinigungen wird entfernt und das Produkt in Vakuum getrocknet.
Ausbeute: ca. 65% hellgelber Feststoff
**¹H-NMR** (CDCl₃): 1.32 (t, 6H, P(O)(OCH₂C*H*₃)₂); 1.44 (s, 9H, C(C*H*₃)₃); 1.75-2.45 (m, 4H, CHC*H*₂C*H*₂P); 3.2-3.85 (m, 4H, NC*H*₂C*H*₂N); 3.73 (s, 3H, OCH₃) ; 4.07 (m, 4H, P(O)(OC*H*₂C*H*₃)₂); 4.28 (m, 1H, NC*H*C(O)); 4.42/4.99 (2d, 2H, NC*H*₂C(O)); 5.22 (s, 2H, OC*H*₂Ph); 5.56 (t, br, 1H, C(O)N*H*CH₂); 7.25 (d, 1H, CC*H*=CHN); 7.38 (s, 5H, *Ph*);7.55 (d, 1H, CCH=CHN).

### Beispiel 8: Herstellung von (R)-2-([2-{N4-Benzyloxycarbonylamino-cytosin-1yl}-acetyl]-[2-tert.butoxycarbonylamino-ethyl]-amino)-4-(diethoxy-phosphoryl)-buttersäure

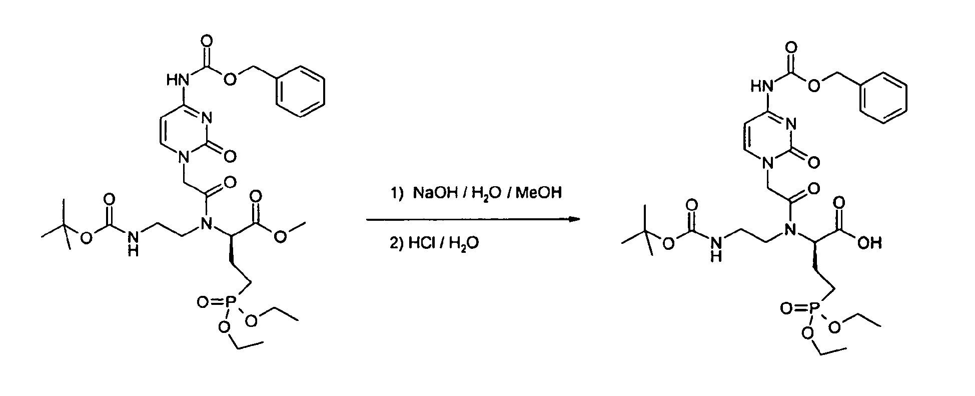

19,1 mmol (R)-2-([2-{N4-Benzyloxycarbonylcytosin-1-yl)-acetyl]-[2-tert.butoxycarbonylamino-ethyl]-amino)-4-(diethoxy-phosphoryl)-buttersäure-methylester wird in 80 ml THF / Wasser (2/3) gelöst und auf 0°C gekühlt. Dazu werden 48 ml einer 1M Lithiumhydroxid-Lösung getropft (pH ~ 9). Der Fortschritt der Reaktion wird mit DC (10% Methanol in Dichlormethan) verfolgt. Nach Abschluss der Reaktion wird die Reaktionslösung mit 130 ml Wasser / NaCl-Lösung verdünnt und einmal mit Dichlormethan (200 ml) extrahiert. Die wässrige Phase wird mit 2M KHSO₄-Lösung auf pH 2-3 eingestellt und mehrfach mit Dichlormethan extrahiert. Dabei wird immer wieder der pH-Wert kontrolliert und nötigenfalls nachkorrigiert. Die vereinigten organischen Phasen werden über MgSO₄ getrocknet, und das Lösungsmittel im Vakuum entfernt. Wenn notwendig, kann das Rohprodukt aus Dichlormethan mit Diethylether umgefällt werden. Schließlich wird das Produkt am Lyophylisator getrocknet.
Ausbeute: ca. 80 % weißgelber Feststoff
**¹H-NMR** (DMSO-d₆): 1.21 (t, 6H, P(O) (OCH₂C*H*₃) ₂) ; 1.39 (s, 9H, C(C*H*₃)₃); 1.70-2.30 (m, 4H, CHC*H*₂C*H*₂P) ; 2.90-3.60 (m, 4H, NC*H*₂C*H*₂N) ; 3.93-4.02 (m, 4H, P(O)(OC*H*₂CH₃)₂) ; 4.25 (m, 1H, NC*H*C(O)); 4.50-4.83 (m, 2H, NC*H*₂C(O)); 5.19 (s, 2H, OC*H*₂Ph) ; 6.88 (m, br, 1H, C(O) N*H*CH₂); 7.02 (d, 1H, CC*H*=CHN) ; 7.31-7.41 (m, 5H, *Ph*); 7.97 (d, 1H, CCH=C*H*N).

### Beispiel 9: Herstellung von weiteren Verbindungen der allgemeinen Formel II

Durch analoge Synthesen wie in den Beispielen 7 und 8 beschrieben, bei denen neben C(Z)-CH₂-COOH weitere Z-geschützte, Benzyl-geschützte (Bzl), Anisoyl-geschützte (An) bzw. Acetyl-geschützte (Ac)und ungeschützte Nukleobasen-Essigsäure-Komponenten A(Z)-CH₂-COOH, A(An)-CH₂-COOH, A(Bzl)-CH₂-COOH oder G(Z)-CH₂-COOH, G(Ac)-CH₂-COOH, C(An)-CH₂-COOH, C(Bzl)-CH₂-COOH, J(Z)-CH₂-COOH, J(Bzl)-CH₂-COOH, J(An)-CH₂-COOH bzw. T-CH₂-COOH (A = Adeninyl, C = Cytosinyl, G = Guaninyl, T = Thyminyl; J = Pseudoisocytosinyl) sowie Phenylessigsäure eingesetzt werden, werden weitere erfindungsgemäße Verbindungen der allgemeinen Formel **II** hergestellt.
**A^{R}(Z) :**
   **¹H-NMR** (CH₃OH-d₄) :1.20 (t, 6H, P(O) (OCH₂C*H*₃) ₂); 1.34 (s, 9H, C(C*H*₃)₃); 1.70-2.30 (m, 4H, CHC*H*₂C*H*₂P); 3.00-3.80 (m, 4H, NC*H*₂C*H*₂N); 3.93-4.02 (m, 4H, P (O)(OC*H*₂C*H*₃)₂); 4.10 (m, 1H, NC*H*C(O)); 5.18 (s, 2H, OC*H*₂Ph); 5.20-5.40 (m, 2H, NC*H*₂C(O)); 7.15-7.40 (m, 5H, *Ph*); 8.14 (s, 1H, N=C*H*N); 8.46 (s, 1H, N=C*H*N) .
**A^{R} (Bzl) :**
   **¹H-NMR** (DMSO-d₆): 1.21 (t, 6H, P(O)(OCH₂C*H*₃) ₂), 1.40 (s, 9H, C(C*H*₃)₃); 1.70-2.20 (m, 4H, CHC*H*₂C*H*₂P,), 2.90-3.75 (m, 4H, NC*H*₂CH₂N); 3.90-4.10 (m, 4H, P(O)(OC*H*₂CH₃)₂); 4.22 (m, 1H, NC*H*C(O)); 5.25-5.45 (m, 2H, NC*H*₂C(O)); 6.96 (m, br, 1H, C(O)N*H*CH₂); 7.50-8.10 (m, 5H, *Ph*); 8.42 (s, 1H, N=C*H*N); 8.69 (s, 1H, N=C*H*N).
**A^{R} (An) :**
   **¹H-NMR** (DMSO-d₆): 1.21 (t, 6H, P(O)(OCH₂C*H*₃) ₂) ; 1.41 (s, 9H, C(C*H*₃) ₃); 1.70-2.20 (m, 4H, CHC*H*₂C*H*₂P) 2.90-3.750 (m, 4H, NC*H*₂C*H*₂N) 3.86 (s, 3H, OC*H*₃) ; 3.90-4.10 (m, 4H, P(O)(OC*H*₂C*H*₃)₂); 4.22 (m, 1H, NC*H*C(O)); 5.25-5.45 (m, 2H, NC*H*₂C(O) ); 6.96 (m, br, 1H, C(O)N*H*CH₂); 7.08 (d, 2H, *Ph*); 8.05 (d, 2H, Ph); 8.42 (s, 1H, N=C*H*N); 8.69 (s, 1H, N=C*H*N) .
**J^{R} (Z) :**
   **¹H-NMR** (DMSO-d₆): 1.32 (t, 6H, P (O) (OCH₂C*H*₃)₂), 1.42 (s, 9H, C (C*H*₃)₃); 1.60-2.50 (m, 4H, CHC*H*₂C*H*₂P,), 3.10-3.55 (m, 4H, NC*H*₂C*H*₂N) ; 3.65-3.90 (m, 2H, NC*H*₂C (O)); 4.00-4.15 (m, 4H, P(O)(OC*H*₂CH₃) ₂) ; 4.20 (m, 1H, NC*H*C(O)) ; 5.24 (s, 2H, OC*H*₂Ph) ; 6.80 (m, br, 1H, C(O)N*H*CH₂); 7.27 (d, 1H, C=C*H*N) ; 7.30-7.50 (m, 5H, *Ph*) .
**J^{R} (An) :**
   **¹H-NMR** (DMSO-d₆): 1.22 (t, 6H, P(O)(OCH₂C*H*₃) ₂) 1.38 (s, 9H, C(C*H*₃)₃); 1.65-2.25 (m, 4H, CHC*H*₂CH₂P); 2.80-3.70 (m, 4H, NC*H*₂C*H*₂N); 2.80-3.70 (m, 2H, CC*H*₂C(O)); 3.84 (s, 3H, OCH₃); 3.90-4.05 (m, 4H, P(O)(OC*H*₂CH₃) ₂); 4.17 (m, 1H, NC*H*C(O)); 6. 81 (m, br, 1H, C(O)N*H*CH₂) ; 7.05 (d, 2H, *Ph*); 7.70 (s, 1H, NC*H*=C); 8.07 (d, 2H, *Ph*) .
**G^{R}(Z) :**
   **¹H-NMR** (DMSO-d₆): 1.18 (t, 6H, P(O) (OCH₂C*H*₃) ₂) , 1.37 (s, 9H, C(C*H*₃)₃); 1.70-2.30 (m, 4H, CHC*H*₂C*H*₂P,), 2.95-3.70 (m, 4H, NC*H*₂C*H*₂N); 3.90-4.10 (m, 4H, P(O)(OC*H*₂CH₃)₂) ; 4.20 (m, 1H, NC*H*C(O)); 4.85-5.20 (m, 2H, NC*H*₂C(O)); 5.269 (s, 2H, OC*H*₂Ph) ; 6.95 (m, br, 1H, C(O)N*H*CH₂) ; 7.30-7.50 (m, 5H, *Ph*); 7.85 (s, 1H, N=C*H*N) .
**G^{R} (Ac) :**
   **¹H-NMR** (DMSO-d₆) : 1.20 (t, 6H, P(O) (OCH₂C*H*₃)₂); 1.41 (s, 9H, C(C*H*₃)₃); 1.70-2.18 (m, 4H, CHC*H*₂C*H*₂P); 2.20 (s, 3H, C*H*₃C(O)); 2.90-3.60 (m, 4H, NC*H*₂C*H*₂N); 3.90-4.10 (m, 4H, P(O)(OC*H*₂C*H*₃) ₂); 4.22 (m, 1H, NC*H*C(O)); 4.91-5.22 (m, 2H, NC*H*₂C(O)); 7.00 (m, br, 1H, C(O)N*H*CH₂) ; 7.88 (s, 1H, N=C*H*-N) ;
**C^{R} (Bzl) :**
   **¹H-NM** (DMSO-d₆): 1.21 (t, 6H, P(O)(OCH₂C*H*₃)₂); 1.40 (s, 9H, C(C*H*₃)₃); 1.70-2.30 (m, 4H, CHC*H*₂CH₂P); 3.20-3.60 (m, 4H, NC*H*₂C*H*₂N); 3.93-4.02 (m, 4H, P(O)(OC*H*₂CH₃)₂) ; 4.28 (m, 1H, NC*H*C(O)); 4.50-4.83 (m, 2H, NC*H*₂C(O)); 6.90 (m, br, 1H, C(O)N*H*CH₂); 7.33 (d, 1H, CC*H*=CHN) ; 7.50-7.55 (m, 2H, *Ph*); 7.62 (d, 1H, CCH=C*H*N); 8.00-8.10 (m, 3H, *Ph*).
**C^{R} (An) :**
   **¹H-NM** (DMSO-d₆): 1.22 (t, 6H, P(O)(OCH₂C*H*₃)₂) ; 1.39 (s, 9H, C(C*H*₃)₃); 1.65-2.10 (m, 4H, CHC*H*₂C*H*₂P); 3.20-3.60 (m, 4H, NC*H*₂C*H*₂N); 3.84 (s, 3H, OC*H*₃); 3.85-4.05 (m, 4H, P(O)(OC*H*₂CH₃)₂) ; 4.25 (m, 1H, NCHC(O)) ; 4.50-4.95 (m, 2H, NC*H*₂C(O)); 6.90 (m, br, 1H, C(O)NHC*H*₂) ; 7.04 (d, 2H, *Ph*); 7.30 (d, 1H, CC*H*=C*H*N); 8.00 (d, 1H, CCH=C*H*N); 8.03 (d, 2H, *Ph*).
**T^{R}:**
   **¹H-NMR** (DMSO-d₆): 1.22 (t, 6H, P(O)(OCH₂C*H*₃)₂); 1.39 (s, 9H, C(C*H*₃)₃) ; 1.65-2.20 (m, 4H, CHC*H*₂C*H*₂P) ; 1.75 (s, 3H, C=CC*H*₃); 2.90-3.50 (m, 4H, NC*H*₂C*H*₂N); 3.90-4.10 (m, 4H, P(O)(OC*H*₂CH₃)₂); 4.18 (m, 1H, NC*H*C(O)); 4.45-4.65 (m, 2H, NC*H*₂C(O)); 6.86 (m, br, 1H, C(O)N*H*CH₂ ); 7.37 (s, 1H, NC*H*=C).
**P^{R}:**
   **¹H-NMR** (DMSO-d₆) 1.20 (t, 6H, P(O)(OCH₂C*H*₃) ₂) ; 1. 38 (s, 9H, C(C*H*₃)₃); 1.46-2.30 (m, 4H, CHC*H*₂C*H*₂P); 3.00-3.45 (m, 4H, NC*H*₂C*H*₂N); 3.50-3.75 (m, 2H, CC*H*₂C(O)); 3.80-4.00 (m, 4H, P(O)(OC*H*₂CH₃)₂); 4.22 (m, 1H, NC*H*C(O)); 7.10-7.30 (m, 5H, Ph).

### Beispiel 10: Herstellung von weiteren Verbindungen der allgemeinen Formel II mit S-Konfiguration am asymmetrischen Zentrum:

Das Herstellungsverfahren für die Verbindungen der allgemeinen Formel **II** mit der R-Konfiguration wird analog für die Herstellung der entsprechenden Verbindungen der allgemeinen Formel **II** mit S-Konfiguration angewendet. Hierbei wird bei der in Beispiel 1 beschriebenen Synthese das (R)-2,5-Dihydro-3,6-dimethoxy-2-isopropylpyrazin als Edukt eingesetzt und die folgenden Synthesen analog wie beschrieben durchgeführt.
**J^{S} (Z):**
   **¹H-NMR** (DMSO-d₆) 1.32 (t, 6H, P (O) (OCH₂C*H*₃) ₂) , 1.42 (s, 9H, C(C*H*₃)₃); 1.60-2.50 (m, 4H, CHC*H*₂C*H*₂P,), 3.10-3.55 (m, 4H, NC*H*₂C*H*₂N) ; 3.65-3.90 (m, 2H, NC*H*₂C(O)); 4.00-4.15 (m, 4H, P(O)(OC*H*₂CH₃)₂) ; 4.20 (m, 1H, NC*H*C(O)); 5.24 (s, 2H, OC*H*₂Ph) ; 6.80 (m, br, 1H, C(O)N*H*CH₂) ; 7.27 (d, 1H, C=C*H*N); 7.30-7.50 (m, 5H, *Ph*).

### Beispiel 11: Allgemeine Synthesevorschrift für erfindungsgemäße Verbindungen:

Durch sequenzielle Verknüpfung von entsprechenden Verbindungen der allgemeinen Formel **II** mit asymmetrischem Zentrum und/oder entsprechender Verbindungen der allgemeinen Formel **II** ohne asymmetrisches Zentrum und/oder Aminosäuren und/oder Aminosäurederivaten und/oder Fluoreszenzmarkern mittels Festphasenpeptidsynthese werden die erfindungsgemäßen Verbindungen hergestellt.

Dabei wird folgendes Syntheseprotokoll verwendet:
Schritt 1: 3 h Vorquellen von 10 mg Harz (Boc-Gly-PAM-MBHA, 0,54 mmol/g) in Dichlormethan.
Schritt 2: Beginn des Synthesezyklus: 4x Waschen mit Dichlormethan.
Schritt 3: Boc-Abspaltung durch Reaktion mit TFA/m-Kresol (95:5). Reaktionsdauer: 2x je 3 min
Schritt 4: 5x Waschen mit Dichlormethan.
Schritt 5: 5x Waschen mit NMP.
Schritt 6: 1 min Voraktivierung von 4 Äquivalenten der entsprechenden geschützten Verbindung der allgemeinen Formel **II** bzw. einer entsprechend geschützten Aminosäure mit 3,8 Äquivalenten HATU und 9 Äquivalenten NMM in NMP/Pyridin (2:1).
Schritt 7: Reaktion der aktivierten geschützten Verbindung der allgemeinen Formel **II** bzw. einer entsprechend geschützten Aminosäure mit der festen Phase (1. Kupplung; Dauer: 30 min).
Schritt 8: 4x Waschen mit NMP.
Schritt 9: 1x Waschen mit Dichlormethan.
Schritt 10: Wiederholung der Schritte 6 bis 8 (2.Kupplung).
Schritt 11: Überprüfung der Kupplungseffizienz mit Ninhydrin (Kaiser-Test; Wenn der Kaiser-Test positiv ist, müssen die Schritte 6 bis 8 mit der entsprechenden geschützten Verbindung der allgemeinen Formel **II** wiederholt werden).
Schritt 12: Nach negativem Kaiser-Test, 2x Capping mit einer Lösung aus Ac₂O/NMP/Pyridin (1:15:25) für je 4 min.
Schritt 13: 5x Waschen mit NMP.
Schritt 14: Wiederholen des Synthesezyklus (Schritte 2 bis 13) bis zur Kupplung mit der letzten entsprechenden geschützten Verbindung der allgemeinen Formel **II.** Anschließend gegebenenfalls Wiederholen des Synthesezyklus (Schritte 2 bis 13) bis zur Kupplung mit der letzten entsprechend geschützten Aminosäure.
Schritt 15: Nach der Kupplung der letzten entsprechenden geschützten Verbindung der allgemeinen Formel **II** bzw. einer entsprechend geschützten Aminosäure werden die Schritte 2 bis 5 zur letzten Boc-Abspaltung und die Schritte 12 und 13 (ohne vorherigen Kaiser-Test) zum abschließenden Capping durchgeführt.
Schritt 16: 5x Waschen mit Dichlormethan.
Schritt 17: zur Trocknung: 5x Waschen mit Diethylether.

Man erhält eine Verbindung der allgemeinen Formel I, die am C-terminalen Ende an das Harz gebunden ist.

### Abspaltung der erfindungsgemäßen Verbindung vom Harz:

Das Harz mit der erfindungsgemäßen Verbindung wird in wässriger Ammoniaklösung (28-30 Gewichtsprozente NH₃ in H₂O) bei 60 °C 20 h gerührt. Das abgespaltene Harz wird anschließend abfiltriert und das Filtrat im Vakuum eingeengt und getrocknet. Das Rohprodukt wird durch präparative HPLC über eine RP-C18-Säule mit Methanol / Wasser gereinigt. Man erhält die erfindungsgemäße Verbindung als farblosen Feststoff in ca. 50%-iger Ausbeute. Die Masse der erfindungsgemäßen Verbindung wird mit MALDI-TOF charakterisiert.

### Beispiel 12: Allgemeine Synthesevorschrift für die erfindungsgemäßen Verbindungen mit Linker:

Durch sequenzielle Verknüpfung von Verbindungen der allgemeinen Formel **II** oder kommerziell erhältlichen ungeschützten bzw. Z-geschützten Verbindungen der allgemeine Formel **II** ohne asymmetrisches Zentrum oder Aminosäuren sowie geeigneten Linker-Monomeren mittels Festphasenpeptidsynthese werden die erfindungsgemäßen Verbindungen mit Linker hergestellt.

### Syntheseprotokoll:

Schritt 1: 3 h Vorquellen von 10 mg Harz (Boc-Gly-PAM-MBHA, 0,54 mmol/g) in Dichlormethan.
Schritt 2: Beginn des Synthesezyklus: 4x Waschen mit Dichlormethan.
Schritt 3: Boc-Abspaltung durch Reaktion mit TFA/m-Kresol (95:5). Reaktionsdauer: 2x je 3 min
Schritt 4: 5x Waschen mit Dichlormethan.
Schritt 5: 5x Waschen mit NMP.
Schritt 6: 1 min Voraktivierung von 4 Äquivalenten der entsprechenden geschützten Verbindung der allgemeinen Formel **II** bzw. einer entsprechend geschützten Aminosäure mit 3,8 Äquivalenten HATU und 9 Äquivalenten NMM in NMP/Pyridin (2:1).
Schritt 7: Reaktion der entsprechenden geschützten Verbindung der allgemeinen Formel **II** bzw. einer entsprechend geschützten Aminosäure mit der festen Phase (1. Kupplung; Dauer: 30 min).
Schritt 8: 4x Waschen mit NMP.
Schritt 9: 1x Waschen mit Dichlormethan.
Schritt 10: Wiederholung der Schritte 6 bis 8 (2.Kupplung).
Schritt 11: Überprüfung der Kupplungseffizienz mit Ninhydrin (Kaiser-Test; Wenn der Kaiser-Test positiv ist, müssen die Schritte 6 bis 8 mit der entsprechenden geschützten Verbindung der allgemeinen Formel **II** wiederholt werden).
Schritt 12: Nach negativem Kaiser-Test, 2x Capping mit einer Lösung aus Ac₂O/NMP/Pyridin (1:15:25) für je 4 min.
Schritt 13: 5x Waschen mit NMP.
Schritt 14: Wiederholen des Synthesezyklus (Schritte 2 bis 13) bis zur Kupplung der Linker eg1 (8-Amino-2,6-dioxaoktansäure).
Schritt 15: Kupplung der Linker: 4x Waschen mit Dichlormethan.
Schritt 16: Boc-Abspaltung durch Reaktion mit TFA/m-Kresol (95:5). Reaktionsdauer: 2x je 3 min
Schritt 17: 5x Waschen mit Dichlormethan.
Schritt 18: 5x Waschen mit NMP.
Schritt 19: 1 min Voraktivierung von 4 Äquivalenten eg1 mit 3,8 Äquivalenten HATU und 9 Äquivalenten NMM in NMP/Pyridin (2:1).
Schritt 20: Reaktion des aktivierten Linkers mit der festen Phase (1. Kupplung; Dauer: 30 min).
Schritt 21: 4x Waschen mit NMP.
Schritt 22: 1x Waschen mit Dichlormethan.
Schritt 23: Wiederholung der Schritte 19 bis 21 (2.Kupplung).
Schritt 24: Überprüfung der Kupplungseffizienz mit Ninhydrin (Kaiser-Test; Wenn der Kaiser-Test positiv ist, müssen die Schritte 19 bis 21 wiederholt werden).
Schritt 25: 2x Capping mit einer Lösung aus Ac₂O/NMP/Pyridin (1:15:25) für je 4 min nach negativem Kaiser-Test.
Schritt 26: 5x Waschen mit NMP.
Schritt 27: 2x Wiederholen des Syntheseabschnitts (Schritte 15 bis 26) für (eg1)₃.
Schritt 28: Wiederholen des Synthesezyklus (Schritte 2 bis 13) bis zur Kupplung mit der letzten entsprechenden geschützten Verbindung der allgemeinen Formel **II.** Anschließend gegebenenfalls Wiederholen des Synthesezyklus (Schritte 2 bis 13) bis zur Kupplung mit der letzten entsprechend geschützten Aminosäure.
Schritt 29: Nach der Kupplung der letzten entsprechenden geschützten Verbindung der allgemeinen Formel **II** bzw. einer entsprechend geschützten Aminosäure werden die Schritte 2 bis 5 zur letzten Boc-Abspaltung und die Schritte 12 und 13 (ohne vorherigen Kaiser-Test) zum abschließenden Capping durchgeführt.
Schritt 30: 5x Waschen mit Dichlormethan.
Schritt 31: zur Trocknung: 5x Waschen mit Diethylether. Man erhält eine erfindungsgemäße Verbindung mit Linker, die am C-terminalen Ende an das Harz gebunden ist.

### Abspaltung der erfindungsgemäßen Verbindung mit Linker vom Harz:

Das Harz mit der erfindungsgemäßen Verbindung mit Linker wird in wässriger Ammoniaklösung (28-30 Gewichtsprozente NH₃ in H₂O) bei 60 °C 20 h gerührt. Das abgespaltene Harz wird anschließend abfiltriert und das Filtrat im Vakuum eingeengt und getrocknet. Das Rohprodukt wird durch präparative HPLC über eine RP-C18-Säule mit Methanol / Wasser gereinigt. Man erhält die erfindungsgemäße Verbindung mit Linker als farblosen Feststoff in 50%-iger Ausbeute. Die Masse der erfindungsgemäßen Verbindung mit Linker wird mit MALDI-TOF charakterisiert.

### Beispiel 13: Weitere Beispiele von Sequenzen

Durch Durchführung der Allgemeinen Synthesevorschrift aus den Beispielen 11 oder 12 werden weitere erfindungsgemäße Verbindungen hergestellt:
Ac-ε(L)TML-A^{R}T^{R}C^{R}C^{R}T^{R}T^{R}C^{R}C^{R}A^{R}G^{R}T^{R}G^{R}G^{R}T^{R}C^{R}-OH
Ac-ε(L)TML-C^{R}C^{R}C^{R}T^{R}C^{R}A^{R}C^{R}T^{R}T^{R}G^{R}A^{R}T^{R}T^{R}T^{R}A^{R}-OH
Ac-ε(L)TML-A^{R}tC^{R}gT^{R}cT^{R}aA^{R}gG^{R}tC^{R}aG^{R}-Gly-NH₂
Ac-ε(L)TML-C^{R}gT^{R}tG^{R}aA^{R}cA^{R}cG^{R}cC^{R}aT^{R}-Gly-NH₂
Ac-ε(L)TML-A^{R}tC^{R}aG^{R}aG^{R}gA^{R}gC^{R}tT^{R}gG^{R}-Gly-NH₂
AC-T^{R}C^{R}G^{R}C^{R}T^{R}G^{R}C^{R}C^{R}A^{R}A^{R}A^{R}G^{R}A^{R}G^{R}T^{R}-OH₂
Ac-ε(L)TML-T^{R}C^{R}G^{R}C^{R}T^{R}G^{R}C^{R}C^{R}A^{R}A^{R}A^{R}G^{R}A^{R}G^{R}T^{R}-ε(L) TML-Gly-NH₂
Ac-ε(L)TML-tC^{R}gC^{R}tG^{R}cC^{R}aA^{R}aG^{R}aG^{R}t-Gly-NH₂
FLu-ε(L)TML-tC^{R}gC^{R}tG^{R}cC^{R}aA^{R}aG^{R}aG^{R}t-Gly-NH₂
Ac-ε(L)TML-cC^{R}tG^{R}tC^{R}tC^{R}tC^{R}aG^{R}tA^{R}c-Gly-NH₂
Ac-ε(L)TML-G^{R}tC^{R}tC^{R}tC^{R}aG^{R}tA^{R}cA^{R}aT^{R}-Gly-NH₂
Ac-ε(L)TML-G^{R}C^{R}T^{R}C^{R}C^{R}T^{R}C^{R}G^{R}C^{R}C^{R}C^{R}T^{R}T^{R}G^{R}C^{R}-ε(L)TML-Gly-NH₂
Ac-ε(L)TML-G^{R}C^{R}T^{R}C^{R}C^{R}T^{R}C^{R}G^{R}C^{R}C^{R}T^{R}T^{R}T^{R}G^{R}C^{R}-ε(L)TML-Gly-NH₂
Ac-ε(L)TML-A^{R}G^{R}C^{R}T^{R}C^{R}C^{R}T^{R}C^{R}G^{R}C^{R}C^{R}C^{R}T^{R}T^{R}G^{R}C^{R}-Gly-NH₂
Ac-ε(L)TML-ε(L) TML-ε(L) TML-ε(L) TML-tC^{R}aC^{R}cA^{R}tG^{R}gT^{R}gG^{R}c^{R}aC^{R}-Gly-NH₂
Ac-ε(L)TML-ε(L)TML-ε(L)TML-ε(L)TML-aG^{R}CT^{R}cC^{R}tC^{R}gC^{R}cC^{R}tT^{R}gC^{R}-Gly-NH₂
Ac-ε(L) TML-ε(L)TML-ε(L)TML-ε(L)TML-tG^{R}gT^{R}c^{R}gG^{R}gT^{R}aG^{R}cG^{R}gC^{R}-Gly-NH₂
Ac-ε(L)TML-ε(L)TML-ε(L)TML-ε(L)TML-cT^{R}gC^{R}aC^{R}gC^{R}tG^{R}CC^{R}gT^{R}cC^{R}-Gly-NH₂
Ac-ε(L)TML-ε(L)TML-ε(L)TML-ε(L)TML-gT^{R}tC^{R}tG^{R}cT^{R}gG^{R}tA^{R}gT^{R}gG^{R}-Gly-NH₂
AC-ε(L)TML-C^{R}T^{R}T^{R}C^{R}G^{R}C^{R}A^{R}C^{R}T^{R}C^{R}A^{R}-ε(L)TML-Gly-NH₂
Ac-cT^{R}tC^{R}gC^{R}aC^{R}tC^{R}a-ε(L) TML-Gly-NH₂
Ac-ε(L) TML-cT^{R}tC^{R}gC^{R}aC^{R}tC^{R}a-eg1-eg1-eg1-Gly-NH₂
Ac-ε(L) TML-ε(L) TML-ε(L)TML-ε(L) TML-cT^{R}tC^{R}gC^{R}aC^{R}tC^{R}a-eg1-eg1-eg1-tJ^{S}tJ^{S}-Gly-NH₂
Ac-ε(L)TML-ε(L)TML-ε(L)TML-ε (L)TML-cT^{R}tC^{R}gC^{R}aC^{R}tC^{R}a-eg1-eg1-eg1-tJ^{R}tJ^{R}-Gly-NH₂
Ac-G^{R}C^{R}T^{R}G^{R}C^{R}C^{R}A^{R}A^{R}A^{R}G^{R}A^{R}G^{R}T^{R}-Gly-NH₂
Ac-ε(L)TML-tcgcT^{R}G^{R}C^{R}C^{R}A^{R}A^{R}A^{R}gagt-Gly-NH₂
Ac-ε(L)TML-ε(L)TML-ε(L)TML-ε(L)TML-T^{R}C^{R}G^{R}ctgccaaagA^{R}G^{R}T^{R}-ε (L) TML-Gly-NH₂
Ac-ε(L)TML-tcgctgccaaagA^{R}G^{R}T^{R}-ε (L)TML-Gly-NH₂
Ac-ε(L)TML-T^{R}C^{R}G^{R}ctgccaaagagt-ε (L)TML-Gly-NH₂
Ac-ε(L)TML-ε(L)TML-ε(L)TML-T^{R}C^{R}gctgC^{R}C^{R}aaagaG^{R}T^{R}-Gly-NH₂
Ac-ε(L)TML-T^{R}cgC^{R}tgC^{R}caA^{R}agA^{R}gt-Gly-NH₂
Ac-ε(L)TML-ε(L) TML-T^{R}CgctgcC^{R}aaagag^{R}T^{R}-Gly-NH₂
Ac-ε(L)TML-tcgctgC^{R}C^{R}aaagagt-Gly-NH₂
Ac-ε(L)TML-tcgcT^{R}gccaaA^{R}gagt-Gly-NH₂
Ac-ε(L)TML-TRcgctgccaaagagT^{R}-Gly-NH₂
Ac-ε(L)TML-tcgctgccaaagagT^{R}-Gly-NH₂
Ac-ε(L)TML-T^{R}cgctgccaaagagt-Gly-NH₂
Ac-ε(L)TML-tcgctgccA^{R}aagagt-Gly-NH₂
Ac-ε(L)TML-gG^{R}cT^{R}cC^{R}CA^{R}aA^{R}gA^{R}tC^{R}tT^{R}-Gly-NH₂
Ac-ε(L)TML-T^{R}cG^{R}gA^{R}gC^{R}cA^{R}gC^{R}cC^{R}cT^{R}t-Gly-NH₂
Ac-ε(L)TML-G^{R}tA^{R}tT^{R}cA^{R}gT^{R}gT^{R}gA^{R}tG^{R}a-Gly-NH₂
Ac-ε(L)TML-gC^{R}tA^{R}tT^{R}aC^{R}cT^{R}tA^{R}aC^{R}cC^{R}-Gly-NH₂
Ac-ε(L)TML-gC^{R}aA^{R}aT^{R}tC^{R}tT^{R}aT^{R}tC^{R}cC^{R}-Gly-NH₂
Ac-ε(L)TML-A^{R}aA^{R}tC^{R}aG^{R}gG^{R}tT^{R}aG^{R}gT^{R}-Gly-NH₂
Flu-ε(L)TML-A^{R}aA^{R}tC^{R}aG^{R}gG^{R}tT^{R}aG^{R}gT^{R}-Gly-NH₂
Ac-ε(L)TML-cG^{R}cC^{R}tT^{R}aT^{R}cC^{R}gT^{R}aG^{R}cC^{R}-Gly-NH₂
Flu-ε(L)TML-cG^{R}CC^{R}tT^{R}aT^{R}cC^{R}gT^{R}aG^{R}cC^{R}-Gly-NH₂
Ac-ε(L)TML-C^{R}gT^{R}gT^{R}cT^{R}gT^{R}gT^{R}tG^{R}tA^{R}g-Gly-NH₂
Ac-ε(L)TML-cA^{R}cG^{R}tA^{R}tG^{R}cT^{R}tC^{R}gT^{R}cT^{R}-Gly-NH₂
Ac-(ε(L)TML)₄-tA^{R}tT^{R}aC^{R}tT^{R}CT^{R}gG^{R}gC^{R}tG^{R}-Gly-NH₂
LiRho-(ε(L)TML)₄-tA^{R}tT^{R}aC^{R}tT^{R}cT^{R}gG^{R}gC^{R}tG^{R}-Gly-NH₂ LiRho = Lissamin Rhodanin B (Sulforhodamin B)
Ac-(ε(L)TML)₄-cT^{R}CT^{R}tG^{R}aT^{R}aA^{R}aT^{R}tT^{R}gA^{R}-Gly-NH₂
LiRho-(ε(L)TML)₄-cT ^{R}cT ^{R}tG^{R}aT^{R}aA^{R}aT^{R}tT^{R}gA^{R}-Gly-NH₂
Ac-(ε(L)TML)₄-tG^{R}gT ^{R}gA ^{R}aA^{R}tT ^{R}gC^{R} tG ^{R}cC^{R}-Gly-NH₂
LiRho-(ε(L)TML)₄-tG^{R}gT^{R}gA^{R}aA^{R}tT^{R}gC^{R}tG^{R}cC^{R}-Gly-NH₂
Ac-(ε(L)TML)₄-gA^{R}gC^{R}tC^{R}tT^{R}cG^{R}tC^{R}gC^{R}tG^{R}-Gly-NH₂
LiRho-(ε(L)TML)₄-gA^{R}gC^{R}tC^{R}tT^{R}cG^{R}tC^{R}gC^{R}tG^{R}-Gly-NH₂
Ac-(ε(L)TML)₄-cT^{R}cC^{R}aT^{R}tA^{R}tC^{R}aT^{R}tC^{R}tC^{R}-Gly-NH₂
LiRho-(ε(L)TML)₄-cT^{R}cC^{R}aT^{R}tA^{R}tC^{R}aT^{R}tC^{R}tC^{R}-Gly-NH₂
Ac-(ε(L)TML)₄-cC^{R}cT^{R}gG^{R}tG^{R}tG^{R}tA^{R}gT^{R}tC^{R}-Gly-NH₂
LiRho-(ε(L)TML)₄-cC^{R}cT^{R}gG^{R}tG^{R}tG^{R}tA^{R}gT^{R}tC^{R}-Gly-NH₂
Ac-(ε(L)TML)₄-A^{R}gC^{R}tcctcG^{R}cC^{R}cT^{R}tG^{R}c-Gly-NH₂
TxRed-(ε(L)TML)₄-A^{R}gC^{R}tcctcG^{R}cC^{R}cT^{R}tG^{R}c-Gly-NH₂
TxRed = Texas Red (Sulforhodamin 101)
Ac-(ε(L)TML)₄-A^{R}gC^{R}tC^{R}cT^{R}cG^{R}cC^{R}cT^{R}tgc-Gly-NH₂ TxRed-(ε(L)TML) ₄-A^{R}gC^{R}tC^{R}cT^{R}cG^{R}cC^{R}cT^{R}tgc-Gly-NH₂ Ac-((L)Lys))₄-G^{R}tA^{R}tT^{R}cA^{R}gtgtgA^{R}tG^{R}a-Gly-NH₂
Ac-(ε(L)TML)₄-G^{R}tA^{R}tT^{R}cA^{R}gtgtgA^{R}tG^{R}a-Gly-NH₂
Ac-(ε(L)TML)₄-gtatT^{R}cA^{R}gtgtgA^{R}tG^{R}a-Gly-NH₂
Ac-(ε(L)TML)₄-cG^{R}cC^{R}tT^{R}atccgT^{R}aG^{R}cC^{R}-Gly-NH₂
Ac-(L-Lys)₄-gC^{R}tA^{R}tT^{R}cccttA^{R}aC^{R}cC^{R}-Gly-NH₂
TxRed- (L-Lys) ₄-gC^{R}tA^{R}tT^{R}accttA^{R}aC^{R}cC^{R}-Gly-NH₂
Ac-(L-Lys)-(L-Lys)-(L-Lys)-(L-Arg)-(L-Lys)-(L-Val)-gC^{R}tA^{R}tT^{R}accttA^{R}aC^{R}cC^{R}-Gly-NH₂
TxRed-(L-Lys)-(L-Lys)-(L-Lys)-(L-Arg)-(L-Lys)-(L-Val)-gC^{R}tA^{R}tT^{R}accttA^{R}aC^{R}cC^{R}-Gly-NH₂
Ac-((L)TML)₄-gC^{R}tA^{R}tT^{R}accttA^{R}aC^{R}cC^{R}-Gly-NH₂
TxRed-(ε(L)TML)₄-gC^{R}tA^{R}tT^{R}accttA^{R}aC^{R}cC^{R}-Gly-NH₂
Ac-(ε(L)TML) ₄-acttG^{R}aA^{R}ttcgtA^{R}tC^{R}c-Gly-NH₂
Ac-(L-Lys)-(L-Lys)-(L-Lys)-(L-Arg)-(L-Lys)-(L-Val)-acttG^{R}aA^{R}ttcgtA^{R}tC^{R}c-Gly-NH₂
Ac-(ε(L)TML)₄-acttG^{R}aattcgtA^{R}tC^{R}C-Gly-NH₂
Ac-(L-Lys)-(L-Lys)-(L-Lys)-(L-Arg)-(L-Lys)-(L-Val)-acttG^{R}aattcgtA^{R}tC^{R}C-Gly-NH₂
Ac-(ε(L)TML) ₄-gtgtA^{R}TA^{R}cacggA^{R}aT^{R}a-Gly-NH₂
Flu-(ε(L)TML) ₄-gtgtA^{R}tA^{R}cacggA^{R}aT^{R}a-Gly-NH₂
Ac-(L-Lys)-(L-Lys)-(L-Lys)-(L-Arg)-(L-Lys)-(L-Val)-gtgtA^{R}tA^{R}cacggA^{A}aT^{R}a-Gly-NH₂
Flu-(L-Lys)-(L-Lys)-(L-Lys)-(L-Arg)-(L-Lys)-(L-Val)-gtgtA^{R}tA^{R}cacggA^{R}aT^{R}a-Gly-NH₂
Ac-(ε(L)TML)₄-gtgtA^{R}tacacggA^{R}aT^{R}a-Gly-NH₂
Flu-(ε(L)TML)₄-gtgtA^{R}tacacggA^{R}aT^{R}a-Gly-NH₂
Ac-(ε(L)TML)₄-ctgcT^{R}gcT^{R}tgctgC^{R}tG^{R}c-Gly-NH₂
Ac-(L-Lys)-(L-Lys)-(L-Lys)-(L-Arg)-(L-Lys)-(L-Val)-ctgcT^{R}gC^{R}tgctgC^{R}tG^{R}c-Gly-NH₂
Ac-(ε(L)TML)₄-ctgcT^{R}gctgctgC^{R}tG^{R}c-Gly-NH₂
Ac-(L-Lys)-(L-Lys)-(L-Lys)-(L-Arg)-(L-Lys)-(L-Val)-ctgcT^{R}gctgctgC^{R}tG^{R}c-Gly-NH₂
Ac-(ε(L)TML)₄-agctC^{R}cT^{R}cggtaG^{R}gT^{R}c-Gly-NH₂
Ac-(L-Lys)-(L-Lys)-(L-Lys)-(L-Arg)-(L-Lys)-(L-Val)-agctC^{R}cT^{R}cggtaG^{R}gT^{R}c-Gly-NH₂
Ac-(ε(L)TML)₄-agctC^{R}ctcggtaG^{R}gT^{R}c-Gly-NH₂
Ac-(L-Lys)-(L-Lys)-(L-Lys)-(L-Arg)-(L-Lys)-(L-Val)-agctC^{R}ctcggtaG^{R}gT^{R}c-Gly-NH₂
Ac-gtccc^{R}tG^{R}aagatG^{R}tC^{R}a-Gly-NH₂
Ac-(ε(L)TML)₄-gtccC^{R}tG^{R}aagatG^{R}tC^{R}a-Gly-NH₂
Ac-gtatT^{R}cA^{R}gtgtgA^{R}tG^{R}a-Gly-NH₂
Ac-(ε(L)TML)₄-gtatT^{R}cA^{R}gtgtgA^{R}tG^{R}a-Gly-NH₂
Ac-gtcgC^{R}tG^{R}tctccG^{R}CT^{R}t-Gly-NH₂
Ac-(ε(L)TML)₄-gtcgC^{R}tG^{R}tctccG^{R}cT^{R}t-Gly-NH₂
Ac-(ε(L)TML)₄-ctccA^{R}tG^{R}gtgctC^{R}aC^{R}t-Gly-NH₂
Ac-(DEPABS)₂-Gly-ctccA^{R}tG^{R}gtgctC^{R}aC^{R}t -Gly-NH₂
Ac-(ε(L)TML)₄-ggctC^{R}cC^{R}aaagaT^{R}cT^{R}t-Gly-NH₂
Ac-(DEPABS)₂-Gly-ggctC^{R}cC^{R}aaagaT^{R}cT^{R}t-Gly-NH₂
Ac-(ε(L)TML)₄-tcggA^{R}gC^{R}cagccC^{R}CT^{R}t-Gly-NH₂
Ac-(DEPABS)₂-Gly-tcggA^{R}gC^{R}cagccC^{R}cT^{R}t-Gly-NH₂
Ac-(ε(L)TML)₄-tcccA^{R}gC^{R}gtgcgC^{R}cA^{R}t-Gly-NH₂
Ac-(DEPABS)₂-Gly-tcccA^{R}gC^{R}gtgcgC^{R}cA^{R}t-Gly-NH₂
Ac-(ε(L)TML)₄-catcC^{R}cA^{R}gcctcC^{R}gT^{R}t-Gly-NH₂
Ac-(DEPABS)₂-Gly-catcC^{R}cA^{R}gcctcC^{R}gT^{R}t-Gly-NH₂
Ac-(ε(L)TML)₄-gtcgC^{R}tG^{R}tctccG^{R}cT^{R}t-Gly-NH₂
Ac-(DEPABS)₂-Gly-gtcgC^{R}tG^{R}tctccG^{R}cT^{R}t-Gly-NH₂
Ac-(L-Lys)-(L-Lys)-(L-Lys)-(L-Arg)-(L-Lys)-(L-Val)-gtcgC^{R}tG^{R}tctccG^{R}cT^{R}t-Gly-NH₂
Ac-(L-Lys)-(L-Lys)-(L-Lys)-(L-Arg)-(L-Lys)-(L-Val)-ctgcT^{R}gC^{R}tgctgC^{R}tG^{R}c-Gly-NH₂
Ac-(DEPABS)₂-Gly-ctgcT^{R}gC^{R}tggctgC^{R}tG^{R}c-Gly-NH₂
Ac-L-Lys)-(L-Lys)-(L-Lys)-(L-Arg)-(L-Lys)-(L-Val)-agctC^{R}CT^{R}cgcccT^{R}tG^{R}C-Gly-NH₂
Ac-(DEPABS)₂-Gly-agctC^{R}cT^{R}cgcccT^{R}tG^{R}c-Gly-NH₂
Ac-(L-Lys)-(L-Lys)-(L-Lys)-(L-Arg)-(L-Lys)-(L-Val)-gcgtG^{R}tG^{R}ggaagG^{R}cA^{R}g-Gly-NH₂
Ac-(DEPABS)₂-Gly-gcgtG^{R}tG^{R}ggaagG^{R}cA^{R}g-Gly-NH₂
DOTA-ε(L)TML-C^{R}aG^{R}tT^{R}aG^{R}gG^{R}tT^{R}aG^{R}-Gly-NH₂

### Beispiel 14: Stärkerer Antisense-Effekt von erfindungsgemäßen Verbindungen gegenüber stereochemisch uneinheitlichen Oligomeren.

H9 Zellen, die chronisch mit HIV-1-NL4-3-Stamm infiziert sind, werden zweimal mit einem PBS-Puffer gewaschen, um bereits produzierte Viren zu entfernen. Die Zellen werden in eine 96-Well-Platte mit 104 Zellen/Well, 4 Wells pro Probe, in 200 µl Kultur-Medium mit oder ohne erfindungsgemäßer Verbindung beziehungsweise Ritonavir bei einer Konzentration inkubiert. Nach 5 Tagen werden von jedem der 4 Wells 40 µl der Zellsuspension entnommen und mit 40 µl Nonidet P-40 inaktiviert.

Die Menge an p24 GAG Antigen wird per quantitativen ELISA-Asssay (Sandwich-ELISA-Biotin-Strepavidin-HRP) nach der Standardmethode des Institutes für Angewandte Mikrobiologie Wien bestimmt. Als Standard werden Proben mit bekannter p24-Menge verwendet, um eine Standard-Kurve zu ermitteln.

Hierbei konnte bei den stereochemisch uneinheitlichen Oligomeren der Sequenz TCGCTGCCAAAGAGT-NH2 eine Reduktion der p24-Menge um 21% gegenüber der unbehandelten Probe festgestellt werden. Die kürzeren, erfindungsgemäßen Verbindungen der Sequenz T^{R}A^{R}G^{R}A^{R}G^{R}C^{R}T^{R}T^{R}C^{R}C^{R}-NH₂ zeigten eine erhöhte Reduktion um 36%.

### Beispiel 15: Wirksamkeit gegen HIV über 2 Tochtergenerationen.

Es wurden Humane CD4⁺ T-Lymphozyten (M8166) 24 Stunden lang mit den erfindungsgemäßen Verbindungen vorinkubiert und anschließend mit HIV-infiziert (Erst-Infektion).

Nach 6 Tagen wurde der Überstand mit den neu gebildeten HI-Viren durch Zentrifugieren von den Zellen abgetrennt. Der Überstand wurde so weit verdünnt, wie notwendig ist, um bei der Folge-Infektion (Zweit-Infektion) eine HIV-Vermehrung in einen nach der Standardmethode messbaren Bereich (ca. 1/5000) zu erhalten. Anschließend wurden nicht infizierte Zellen (M8166) mit HIV aus dem verdünnten Überstand infiziert und die p24-Menge nach weiteren 6 Tagen mit Hilfe eines quantitativen p24-Elisa gemessen.

In dem Experiment wurden erfindungsgemäßen Verbindungen mit einer wirksamen HIV-Sequenz (Match), welche aufgrund ihrer Sequenz an HIV-RNA binden können, verwendet. Weiterhin wurden erfindungsgemäßen Verbindungen eingesetzt, deren Sequenz keine Übereinstimmung für eine Bindung an die HIV-RNA aufweist (No-Match). Die erhaltenen Messwerte aus dem quantitativen p24-Elisa nach der Zweit-Infektion wurden mit den ebenso erhaltenen Messwerten aus der Positiv-Kontrolle, bei der keine erfindungsgemäßen Verbindungen eingesetzt wurden, ins Verhältnis gesetzt und sind in der folgenden Tabelle zusammengestellt.

| Keine erfindungsgemäßen Verbindungen (Positiv-Kontrolle) | | | | | |
|---|---|---|---|---|---|
| P24 [%] | | | 100 | | |
| Erfindungsgemäße Verbindungen (Match): | | | | | |
| Ac-ε(L)TML-T^{R}C^{R}G^{R}C^{R}T^{R}G^{R}C^{R}C^{R}A^{R}A^{R}A^{R}G^{R}A^{R}G^{R}T^{R}-ε(L)TML-Gly-NH₂ | | | | | |
| Konzentration[µM) | 25 | 12,5 | 6, 25 | 3,125 | 1,5625 |
| P24 [%] | 18 | 20 | 73 | 48 | 68 |
| Erfindungsgemäße Verbindungen (No-Match): | | | | | |
| Ac-ε(L)TML-G^{R}C^{R}T^{R}C^{R}C^{R}T^{R}C^{R}G^{R}C^{R}C^{R}C^{R}T^{R}T^{R}G^{R}C^{R}-ε(L)TML-Gly-NH₂ | | | | | |
| Konzentration[µM] ) | 25 | 12,5 | 6, 25 | 3,125 | 1,5625 |
| P24 [%] | 64 | 84 | 84 | 71 | 90 |

Nach der Zweit-Infektion ist jedoch bei den erfindungsgemäßen Verbindungen mit HIV-Sequenz bei den beiden höchsten eingesetzten Konzentrationen eine deutliche Reduktion des p24-Gehalts zu erkennen.

### Beispiel 16: Anreicherung von erfindungsgemäßen Verbindungen in Zellen mit Target-Sequenz

Die erfindungsgemäßen Verbindungen reichern sich überraschend stark in Zellen an, in denen eine komplementäre DNA- bzw. RNA-Sequenz vorhanden ist.

In einem entsprechenden Experiment wurden erfindungsgemäße Verbindungen mit einer HIV-Sequenz, welche an eine komplementäre DNA- bzw. RNA-Sequenz binden können, mit Fluorescein (Flu) gelabelt. Mit diesem gelabelten erfindungsgemäßen Verbindungen wurden einmal HIV-infizierte Humane CD4⁺ T-Lymphozyten (M8166) eine einmal nicht infizierte Humane CD4⁺ T-Lymphozyten (M8166) inkubiert.

Die FACS-Analyse der jeweiligen Zellen zeigt eine wesentlich stärkere Anreicherung der erfindungsgemäßen Verbindungen mit HIV-Sequenz in den HIV-infizierten Zellen, wo eine komplementäre DNA- bzw. RNA-Sequenz vorhanden ist.

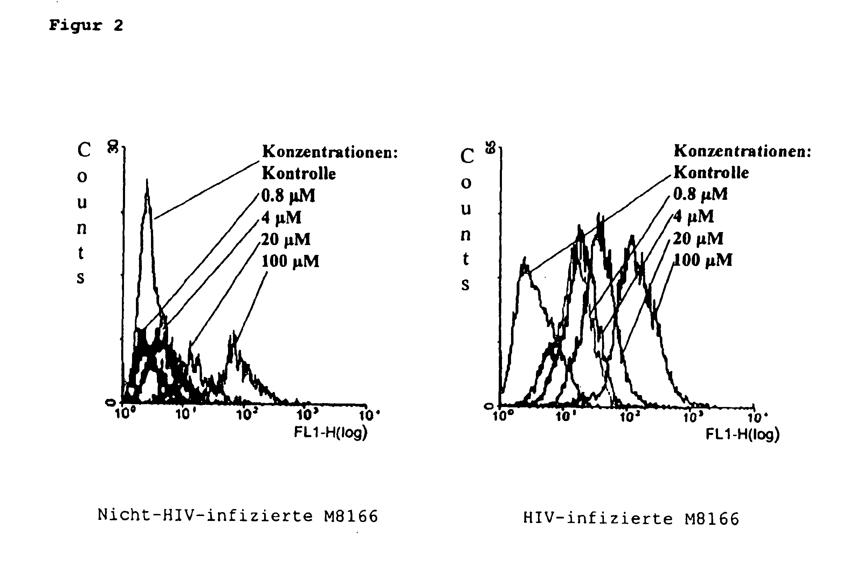

In Figur 2 sind die entsprechenden Ergebnisse unter Verwendung der erfindungsgemäßen Verbindung Flu-εTML-T^{R}C^{R}G^{R}C^{R}T^{R}G^{R}C^{R}C^{R}A^{R}A^{R}A^{R}G^{R}A^{R}G^{R}T^{R}-Gly-NH₂ dargestellt.

### Beispiel 17: Vergleich der Zellgängigkeit verschiedener erfindungsgemäßer Verbindungen

Mit dem gleichen Versuchaufbau wie im Beispiel 16 wurde die Zellgängigkeit von erfindungsgemäßen Verbindungen, bei denen sich an jedem asymmetrischen Zentrum ein Rest substituierter Rest R¹ befindet, mit der Zellgängigkeit von erfindungsgemäßen Verbindungen, bei denen an jedem zweiten asymmetrischen Zentrum der Rest R¹ durch ein H-Atom ersetzt worden ist, verglichen.

Die FACS-Analysen der jeweiligen Zellen zeigen keinen Unterschied in der Zellgängigkeit zwischen den beiden erfindungsgemäßen Verbindungen.

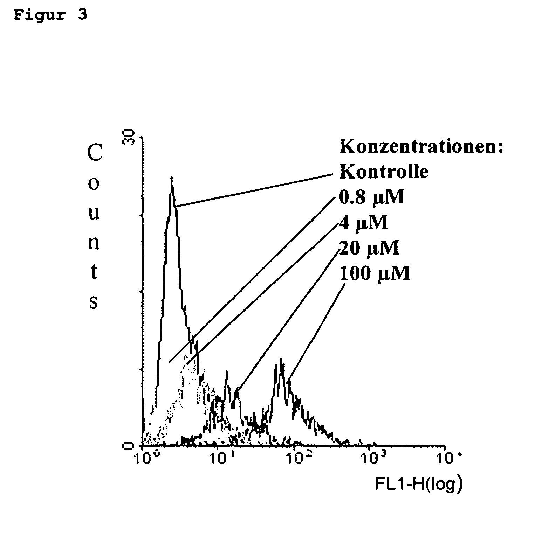

In Figur 3 sind die entsprechenden Ergebnisse unter Verwendung der erfindungsgemäßen Verbindung Flu-εTML-T^{R}C^{R}G^{R}C^{R}T^{R}G^{R}C^{R}C^{R}A^{R}A^{R}A^{R}G^{R}A^{R}G^{R}T^{R}-Gly-NH₂ dargestellt.

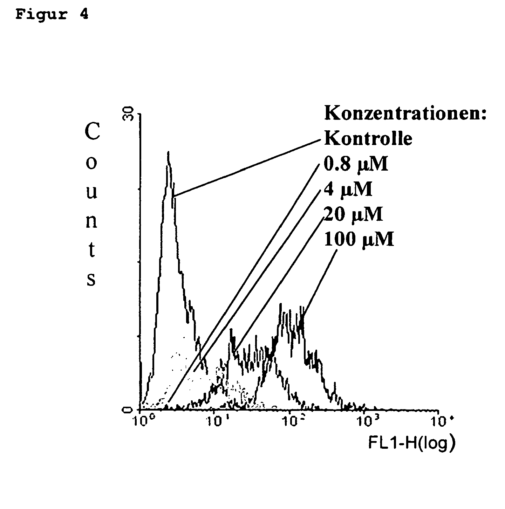

In Figur 4 sind die entsprechenden Ergebnisse unter Verwendung der erfindungsgemäßen Verbindung Flu-εTML-tC^{R}gC^{R}tG^{R}cC^{R}aA^{R}aG^{R}aG^{R}t-Gly-NH₂ dargestellt.

### Beispiel 18: Nachweis der erfindungsgemäßen Verbindungen im Gewebe des Magen-Darm-Trakts und der Schwimmblase von Medaka-Fischen

Medaka-Fische wurden zwei Tage in einer 100µM Lösung der erfindungsgemäßen Verbindung TxRed-(εTML)₄-A^{R}GC^{R}TC^{R}CT^{R}CG^{R}CC^{R}CT^{R}TGC-Gly-NH₂ gehalten und dann in frisches Wasser umgesetzt. Anschließend wurde die Verteilung der erfindungsgemäßen Verbindung innerhalb der Fische am Tag 1, sowie an den Tagen 2 und 5 nach der Umsetzung in frisches Wasser unter dem Fluoreszenz-Mikroskop untersucht. Die Bilder zeigen, dass selbst nach 5 Tagen die erfindungsgemäßen Verbindungen noch im Magen-Darm-Takt nachweisbar sind.

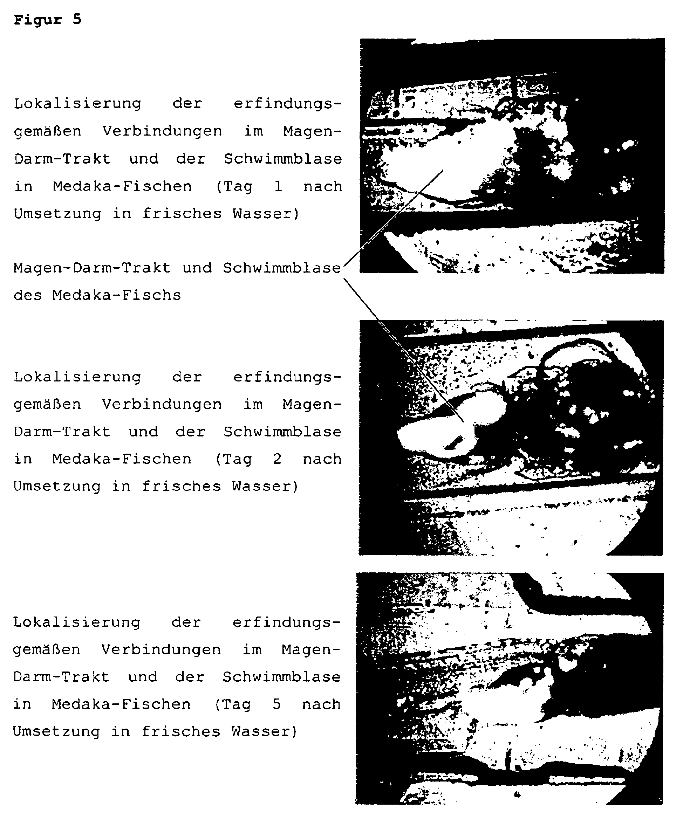

Figur 5 zeigt die entsprechenden Fluoreszenz-mikroskopischen Aufnahmen.

### Beispiel 19: In-vivo-Reduktion von Cholesterin, ApoB100 und ApoB48 in Mäusen durch intravenöse Behandlung mit verbindungsgemälßen Verbindungen

Die erfindungsgemäße Verbindung Ac-(εTML)₄-gtatT^{R}cA^{R}gtgtgA^{R}tG^{R}a-Gly-NH₂, die eine Match-Sequenz zur Target-Sequenz von ApoB100 darstellt, wurde auf ihre pharmakologische Wirksamkeit in Mäusen untersucht. Als Negativ-Kontrolle diente hierbei die Injektion von reinem PBS-Puffer. Dabei wurden den Mäusen an drei aufeinander folgenden Tagen drei verschiedene Konzentrationen ((25 mg kg⁻¹, 12.5 mg kg⁻¹, 6.25 mg kg⁻¹) der effektiven erfindungsgemäßen Verbindung (gelöst in PBS-Puffer) und der Kontrolle in Portionen von 0,1 ml einmal täglich intravenös verabreicht. Am vierten Tag wurden Blutproben der Mäuse genommen und auf Ihren Gehalt an Cholesterin, ApoB100 und ApoB48 untersucht. Die Werte der folgenden Tabelle geben den Gehalt von Cholesterin und ApoB100 im Verhältnis zu den nur mit PBS behandelten Mäusen an.

| Erfindungsgemäße Verbindung: | | | |
|---|---|---|---|
| Ac-(εTML)₄-gtatT^{R}cA^{R}gtgtgA^{R}tG^{R}a-Gly-NH₂ | | | |
| Konzentration[mg/kg] | 25 | 12,5 | 6, 25 |
| Cholesterin [%] | 75 | 85 | 98 |
| ApoB 100[%] | 59 | 67 | -- |
| ApoB 48 [%] | 68 | 74 | 80 |

Die Ergebnisse zeigen eine deutliche und konzentrationsabhängige Reduktion von Cholesterin, ApoB100 und ApoB48 im Blut der Mäuse.

### Beispiel 20: Wirksamkeit der erfindungsgemäßen Verbindungen gegen Krebs

Die erfindungsgemäße Verbindung Ac-εTML-T^{R}cG^{R}gA^{R}gC^{R}cA^{R}gC^{R}cC^{R}cT^{R}t-Gly-NH₂, die eine Match-Sequenz zur Target-Sequenz zu Her2/neu darstellt, wurde auf proliferationhemmende Wirkung der Her2/neu überexprimierende Zellinie MDA453 untersucht. Als negative Kontrolle diente dabei die erfindungsgemäße Verbindung Ac-εTML-cG^{R}cC^{R}tT^{R}aT^{R}CC^{R}gT^{R}aG^{R}cC^{R}-Gly-NH₂ die keine Match-Sequenz zur Target-Sequenz zu Her2/neu darstellt, sowie nicht behandelte MDA453 Kontrollzellen.

Zwischen 5000 und 10000 MDA453 Zellen werden am Tag 1 in 96 Well-Platten ausgesät. Am Tag 2 werden die erfindungsgemäße Match-Sequenz beziehungsweise die erfindungsgemäße Kontroll-Sequenz in Konzentration von jeweils 1 µM zugefügt. An den Tagen 3, 4 und 5 wird das Zellmedium ausgetauscht und durch frisches Medium, welches die erfindungsgemäßen Verbindungen in jeweils 1 µM Konzentration enthält, ersetzt. Die proliferationshemmende Wirkung wird durch Bestimmung des DNA-Gehalts in den einzelnen Wells mittels Propidium-Iodid bestimmt.

| Keine erfindungsgemäßen Verbindung (Negativ-Kontrolle) | |
|---|---|
| DNA [%] | 100 |
| Erfindungsgemäße Verbindung (Match): | |
| Ac-εTML-T^{R}cG^{R}gA^{R}gC^{R}cA^{R}gC^{R}cC^{R}CT^{R}t-Gly-NH₂ | |
| DNA [%] | 67 |
| Erfindungsgemäße Verbindung (No-Match): | |
| Ac-εTML-cG^{R}cC^{R}tT^{R}aT^{R}cC^{R}gT^{R}aG^{R}cC^{R}-Gly-NH₂ | |
| DNA [%] | 103 |

## Patentansprüche

1. Verbindung der Formel I, 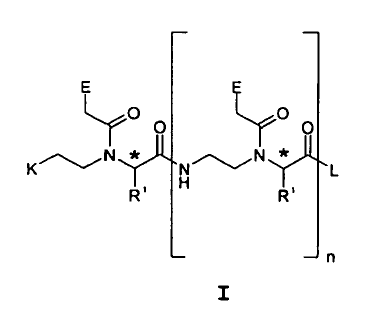 wobei
n eine ganze Zahl von 7 bis 35 ist,
jedes E unabhängig voneinander ein H-Atom, ein substituierter oder unsubstituierter Phenylrest, ein substituierter oder unsubstituierter Heterocyclus, eine gegebenenfalls mit Schutzgruppen substituierte Nukleobase, oder ein DNA-Intercalator ist,
jedes R¹ unabhängig voneinander ein H-Atom oder eine Seitenkette einer natürlichen oder nichtnatürlichen Aminosäure, oder ein gegebenenfalls substituierter Alkyl-, Alkenyl-, Alkylaryl-, Aryl-, heterocyclischer oder alicyclischer Rest mit bis zu 20 C-Atomen ist, wobei mindestens einer der gegebenenfalls substituierten Alkyl-, Alkenyl-, Alkylaryl-, Aryl-, oder alicyclischen Reste mit bis zu 20 C-Atomen mit einer oder mehreren Phosphonsäureester- oder Phosphonsäure-Funktionen substituiert ist,
K eine Gruppe der Formel -NR²R³, -N^{⊕}R²R³R⁴, -NR²(CO)R³ oder - NR²(CS)R³ ist, wobei R², R³ und R⁴ unabhängig voneinander ein H-Atom, ein Alkylrest, Amino-Schutzgruppe, Reporterligand, Fluoreszenz-Marker, Intercalator, Chelator, Aminosäure, Peptid, Protein, Kohlenhydrat, Lipid, Steroid, Fettsäure, Oligonukleotid, Quantum-Dot, FRET-Quencher oder ein in Wasser lösliches oder unlösliches Polymer sind, wobei jeder der vorstehenden Reste gegebenenfalls substituiert sein kann,
L eine Gruppe der Formel -NR⁵R⁶, -NR⁵(CO)R⁶, -NR⁵(CS)R⁶, -OR⁷ oder -SR⁷ ist, wobei R⁵ und R⁶ unabhängig voneinander ein H-Atom, ein Alkylrest, Reporterligand, Fluoreszenz-Marker, Intercalator, Chelator, Aminosäure, Aminosäureamid, Peptid, Peptidamid, Protein, Kohlenhydrat, Lipid, Steroid, Fettsäure, oligonukleotid, Quantum-Dot, FRET-Quencher oder ein in Wasser lösliches oder unlösliches Polymer sind, wobei jeder der vorstehenden Reste gegebenenfalls substituiert sein kann und R⁷ ein H-Atom ein Alkylrest, Reporterligand, Fluoreszenz-Marker, Intercalator, Chelator, Aminosäure, Aminosäureamid, Peptid, Peptidamid, Protein, Kohlenhydrat, Lipid, Steroid, Fettsäure, Oligonukleotid, Quantum-Dot, FRET-Quencher oder ein in Wasser lösliches oder unlösliches Polymer ist, wobei jeder der vorstehenden Reste gegebenenfalls substituiert sein kann,
wobei die Verbindung nach Formel I mindestens 2 asymmetrische Zentren aufweist, und
wobei mindestens 70% der Anzahl der asymmetrischen Zentren, die Reste mit einer oder mehreren Phosphonsäureester- oder Phosphonsäure-Funktionen aufweisen, die R-Konfiguration oder die S-Konfiguration haben, wenn in der Verbindung der allgemeinen Formel 1 2 bis 36 asymmetrische Zentren und 1 bis 36 gegebenenfalls substitutierte Reste R¹ mit einer oder mehreren Phosphonsäureester- oder Phosphonsäure-Funktionen vorhanden sind.

2. Verbindung nach einem der vorhergehenden Ansprüche, wobei jeder zweite Rest R¹ oder jeder dritte Rest R¹ unabhängig voneinander ein gegebenenfalls substituierter Alkyl-, Alkenyl-, Alkylaryl-, Aryl- oder alicyclischer Rest mit bis zu 20 C-Atomen ist und die übrigen Reste R¹ H-Atome sind.

3. Verbindung nach einem der vorstehenden Ansprüche, wobei zwei, drei oder mehr benachbarte Reste R¹ unabhängig voneinander gegebenenfalls substituierte Alkyl-, Alkenyl-, Alkylaryl-, Aryl- oder alicyclischer Reste mit bis zu 20 C-Atomen sind und die übrigen Reste R¹ H-Atome sind.

4. Verbindung nach Anspruch 1, wobei jedes R¹ unabhängig voneinander ein gegebenenfalls substituierter Alkyl-, Alkenyl-, Alkylaryl-, Aryl- oder alicyclischer Rest mit bis zu 20 C-Atomen ist.

5. Verbindung nach einem der vorhergehenden Ansprüche, wobei einer oder mehrere der Reste R¹ unabhängig voneinander Phosphonsäureester- oder Phosphonsäure-Funktionen aufweist.

6. Verbindung nach einem der vorhergehenden Ansprüche, wobei alle asymmetrischen Zentren die gleiche Konfiguration aufweisen.

7. Verbindung nach einem der vorhergehenden Ansprüche, wobei alle asymmetrischen Zentren (S)-Konfiguration aufweisen.

8. Verbindung nach einem der Ansprüche 1 bis 6, wobei alle asymmetrischen Zentren (R)-Konfiguration aufweisen.

9. Verbindung nach einem der vorhergehenden Ansprüche, wobei jedes R¹ unabhängig voneinander eine oder mehrere Phosphonsäureester- oder Phosphonsäure-Funktionen aufweist, wobei die Phosphonsäureester- Funktionen die Formel -P(=O)(OV)₂ oder -P(=O)(OV)(OH) besitzen, wobei jedes V unabhängig voneinander ein unsubstituierter Alkyl-, Alkenyl-, Alkylaryl-, Aryl-, oder alicyclischer Rest mit bis zu 20 C-Atomen ist.

10. Verbindung nach Anspruch 9, wobei jedes V unabhängig voneinander ein Methyl-, Ethyl-, Cyclohexyl oder Benzyl-Rest ist.

11. Verbindung, die mindestens zwei Verbindungen nach einem der Ansprüche 1 bis 10 enthält, welche über einen Linker miteinander verbunden sind, wobei der Linker insbesondere eine Alkylkette, ein Peptid, ein Oligonukleotid oder ein Oligomer ist, das aus mindestens drei 8-Amino-3,6-dioxaoctansäure-Einheiten aufgebaut ist.

12. Zusammensetzung, die mindestens eine Verbindung nach einem der vorstehenden Ansprüche enthält.

13. Pharmazeutische Zusammensetzung, die mindestens eine Verbindung oder Zusammensetzung nach einem der Ansprüche 1 bis 12, gegebenenfalls in Kombination mit mindestens einem Träger, Lösungsmittel oder sonstigen pharmazeutischen Hilfsstoff, enthält.

14. Verwendung einer Verbindung oder Zusammensetzung nach einem der Ansprüche 1 bis 12 oder einer pharmazeutischen zusammensetzung nach Anspruch 13 für die Herstellung eines Arzneimittels zur Behandlung von Viruserkrankungen, Krebs, Entzündungskrankheiten, neurologischen Erkrankungen, Erkrankungen des Magen-Darm-Traktes oder metabolischen Erkrankungen, insbesondere zur Behandlung von HIV, AIDS oder Hepatitis, oder zur Behandlung von Hautkrebs, Lungenkrebs, Leberkrebs, Prostatakrebs, Leukämie oder Gehirntumoren, oder zur Behandlung von Asthma oder Schuppenflechte, oder zur Behandlung der Parkinson-Krankheit, oder zur Behandlung von Darmkrebs, Morbus Crohn oder Fettleibigkeit, oder zur Behandlung von Krankheiten, die mit einem erhöhten Cholesterinspiegel im Blut zusammenhängen.

## Claims

1. Compound of the formula **I**, 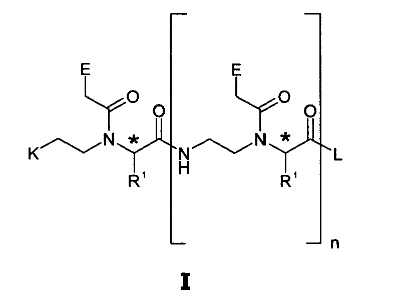 wherein
n represents an integer from 7 to 35,
each E independently of each other represents a hydrogen atom, a substituted or unsubstituted phenyl rest, a substituted or unsubstituted heterocycle, a nucleobase, optionally substituted with protecting groups, or a DNA intercalator,
each R¹ independently of each other represents a hydrogen atom or a side chain of a naturally occurring or non-naturally occurring amino acid, or an optionally substituted alkyl, alkenyl, alkylaryl, aryl, heterocyclic or alicyclic rest having up to 20 carbon atoms, wherein at least one of the optionally substituted alkyl, alkenyl, alkylaryl, aryl, heterocyclic or alicyclic rests having up to 20 carbon atoms is substituted with one or more phosphonic acid ester functions or phosphononic acid functions,
K represents a group of the formula -NR²R³, -N^{⊕}R²R³R⁴, -NR²(CO)R³ or -NR²(CS)R³, wherein R², R³ and R⁴ independently of each other represent a hydrogen atom, an alkyl rest, amino protecting group, reporter ligand, fluorescence marker, intercalator, chelator, amino acid, peptide, protein, carbohydrate, lipid, steroid, fatty acid, oligonucleotide, quantum dot, FRET quencher (fluorescence resonance energy transfer quencher) or a polymer soluble or insoluble in water, wherein each of the above mentioned rests optionally may be substituted,
L represents a group of the formula -NR⁵R⁶, -NR⁵(CO)R⁶, -NR⁵(CS)R⁶, -OR⁷ or -SR⁷, wherein R⁵ and R⁶ independently of each other represent a hydrogen atom, an alkyl rest, reporter ligand, fluorescence marker, intercalator, chelator, amino acid, amino acid amide, peptide, peptide amide, protein, carbohydrate, lipid, steroid, fatty acid, oligonucleotide, quantum dot, FRET quencher (fluorescence resonance energy transfer quencher) or a polymer soluble or insoluble in water, wherein each of the above mentioned rests optionally may be substituted, and wherein R⁷ represents a hydrogen atom, an alkyl rest, reporter ligand, fluorescence marker, intercalator, chelator, amino acid, amino acid amide, peptide, peptide amide, protein, carbohydrate, lipid, steroid, fatty acid, oligonucleotide, quantum dot, FRET quencher or a polymer soluble or insoluble in water, wherein each of the above mentioned rests optionally may be substituted,
wherein the compound of the formula **I** has at least two asymmetric centers, and
wherein at least 70% of the number of the asymmetric centers having rests with one or more phosphonic acid ester functions or phosphonic acid functions exhibit the R configuration or the S configuration, if 2 to 36 asymmetric centers and 1 to 36 optionally substituted rests R¹ having one or more phosphonic acid ester functions or phosphonic acid functions are present in the compound of the general formula **I**.

2. Compound according to one of the preceding claims, wherein each second rest R¹ or each third rest R¹ independently of each other represents an optionally substituted alkyl, alkenyl, alkylaryl, aryl or alicyclic rest having up to 20 carbon atoms, and the remaining rests R¹ represent hydrogen atoms.

3. Compound according to one of the preceding claims, wherein two, three or more adjacent rests R¹ independently of each other represent optionally substituted alkyl, alkenyl, alkylaryl, aryl or alicyclic rests having up to 20 carbon atoms, and the remaining rests R¹ represent hydrogen atoms.

4. Compound according to claim 1, wherein each R¹ independently of each other represents an optionally substituted alkyl, alkenyl, alkylaryl, aryl or alicyclic rest having up to 20 carbon atoms.

5. Compound according to one of the preceding claims, wherein one or more of the rests R¹ independently of each other has phosphonic acid ester functions or phosphonic acid functions.

6. Compound according to one of the preceding claims, wherein all asymmetric centers have the same configuration.

7. Compound according to one of the preceding claims, wherein all asymmetric centers have a (S) configuration.

8. Compound according to claims 1 to 6, wherein all asymmetric centers have a (R)-configuration.

9. Compound according to one of the preceding claims, wherein each rest R¹ independently of each other has one or more phosphonic acid ester functions or phosphonic acid functions, wherein the phosphonic acid ester functions posess the formula -P(=O)(OV)₂ or -P(=O)(OV)(OH), wherein each V independently of each other represents an unsubstituted alkyl, alkenyl, alkylaryl, aryl, or alicyclic rest having up to 20 carbon atoms.

10. Compound according to claim 9, wherein each V independently of each other represents a methyl, ethyl, cyclohexyl or benzyl rest.

11. Compound containing at least two compounds according to one of the claims 1 to 10 which are bound to each other via a linker, wherein the linker represents an alkyl chain, a peptide, an oligonucleotide, or an oligomer which is composed of at least three 8-amino-3,6-dioxaoctanoic acid units.

12. Composition which contains at least one compound according to one of the preceding claims.

13. Pharmaceutical composition, which contains at least one compound or composition according to one of the claims 1 to 12, optionally in combination with at least one carrier, solvent or other pharmaceutical adjuvant.

14. Use of a compound or a composition according to one of the claims 1 to 12 or a pharmaceutical composition according to claim 13 for the preparation of a medicament for the treatment of virus diseases, cancer, inflammation diseases, neurological diseases, diseases of the gastrointestinal tract or metabolic diseases, especially for the treatment of HIV, AIDS or hepatitis, or for the treatment of skin cancer, lung cancer, liver cancer, prostate cancer, leukemia or brain tumors, or for the treatment of asthma or psoriasis, or for the treatment of Parkinson's disease, or for the treatment of colon cancer, Morbus Crohn or adiposity, or for the treatment of diseases that are correlated with an increased cholesterol concentration in the blood.

## Revendications

1. Composé de formule I, 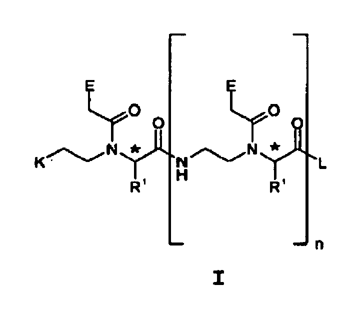 dans laquelle
n est un entier compris entre 7 et 35,
chaque E est, indépendamment l'un de l'autre, un atome de H, un radical phényle substitué ou non substitué, un hétérocycle substitué ou non substitué, une nucléobase le cas échéant substituée avec des groupes protecteurs, ou un intercalateur d'ADN,
chaque R¹ est, indépendamment l'un de l'autre, un atome de H ou une chaîne latérale d'un acide aminé naturel ou non naturel, ou un radical alkyle, alcényle, alkylaryle, aryle, hétérocyclique ou alicyclique le cas échéant substitué comprenant jusqu'à 20 atomes de C, où au moins un des radicaux alkyle, alcényle, alkylaryle, aryle ou alicycliques le cas échéant substitués, comprenant jusqu'à 20 atomes de C, est substitué par un ou plusieurs groupes fonctionnels ester d'acide phosphonique ou acide phosphonique,
K est un groupe de formule -NR²R³_{,} -N^{⊕}R²R³R⁴, -NR²(CO)R³ ou -NR²(CS)R³, où R², R³ et R⁴ sont, indépendamment les uns des autres, un atome de H, un radical alkyle, un groupe amino-protecteur, un ligand rapporteur, un marqueur de fluorescence, un intercalateur, un chélateur, un acide aminé, un peptide, une protéine, un hydrate de carbone, un lipide, un stéroïde, un acide gras, un oligonucléotide, un point quantique (*quantum dot*), un atténuateur (*quencher*) FRET ou un polymère soluble ou insoluble dans l'eau, chacun des radicaux précédents pouvant être le cas échéant substitué,
L est un groupe de formule -NR⁵R⁶, -NR⁵(CO)R⁶ ou -NR⁵(CS)R⁶, -OR⁷ ou -SR⁷, où R⁵ et R⁶ sont, indépendamment l'un de l'autre, un atome de H, un radical alkyle, un ligand rapporteur, un marqueur de fluorescence, un intercalateur, un chélateur, un acide aminé, un aminoamide, un peptide, un amide peptidique, une protéine, un hydrate de carbone, un lipide, un stéroïde, un acide gras, un oligonucléotide, un point quantique (*quantum dot),* un atténuateur (*quencher*) FRET ou un polymère soluble ou insoluble dans l'eau, chacun des radicaux précédents pouvant être le cas échéant substitué, et R⁷ est un atome de H, un radical alkyle, un ligand rapporteur, un marqueur de fluorescence, un intercalateur, un chélateur, un acide aminé, un aminoamide, un peptide, un amide peptidique, une protéine, un hydrate de carbone, un lipide, un stéroïde, un acide gras, un oligonucléotide, un point quantique (*quantum dot*), un atténuateur (*quencher*) FRET ou un polymère soluble ou insoluble dans l'eau, chacun des radicaux précédents pouvant être le cas échéant substitué,
dans lequel le composé selon la formule I comprend au moins 2 centres asymétriques, et
dans lequel au moins 70 % du nombre des centres asymétriques qui présentent des radicaux comprenant un ou plusieurs groupes fonctionnels ester d'acide phosphonique ou acide phosphonique, ont une configuration R ou une configuration S, quand, dans le composé de formule générale I, 2 à 36 centres asymétriques et 1 à 36 radicaux R¹ le cas échéant substitués sont présents avec un ou plusieurs groupes fonctionnels ester d'acide phosphonique ou acide phosphonique.

2. Composé selon l'une des revendications précédentes, dans lequel chaque deuxième radical R¹ ou chaque troisième radical R¹ est, indépendamment l'un de l'autre, un radical alkyle, alcényle, alkylaryle, aryle ou alicyclique, le cas échéant substitué, comprenant jusqu'à 20 atomes de C, et les autres radicaux R¹ sont des atomes de H.

3. Composé selon l'une des revendications précédentes, dans lequel deux, trois ou plus radicaux voisins R¹ sont, indépendamment les uns des autres, un radical alkyle, alcényle, alkylaryle, aryle ou alicyclique, le cas échéant substitué, comprenant jusqu'à 20 atomes de C, et les autres radicaux R¹ sont des atomes de H.

4. Composé selon la revendication 1, dans lequel chaque R¹ est, indépendamment l'un de l'autre, un radical alkyle, alcényle, alkylaryle, aryle ou alicyclique, le cas échéant substitué, comprenant jusqu'à 20 atomes de C.

5. Composé selon l'une des revendications précédentes, dans lequel un ou plusieurs des radicaux R¹ comprennent, indépendamment les uns des autres, des groupes fonctionnels ester d'acide phosphonique ou acide phosphonique.

6. Composé selon l'une des revendications précédentes, dans lequel tous les centres asymétriques présentent la même configuration.

7. Composé selon l'une des revendications précédentes, dans lequel tous les centres asymétriques présentent une configuration (S).

8. Composé selon l'une des revendications 1 à 6, dans lequel tous les centres asymétriques présentent une configuration (R).

9. Composé selon l'une des revendications précédentes, dans lequel chaque R¹ comprend, indépendamment l'un de l'autre, des groupes fonctionnels ester d'acide phosphonique ou acide phosphonique, où les groupes fonctionnels ester d'acide phosphonique ont la formule -P(=O)(OV)₂, ou -P(=O)(OV)(OH), où chaque V est, indépendamment l'un de l'autre, un radical alkyle, alcényle, alkylaryle, aryle ou alicyclique, non substitué, comprenant jusqu'à 20 atomes de C.

10. Composé selon la revendication 9, dans lequel chaque V est, indépendamment l'un de l'autre, un radical méthyle, éthyle, cyclohexyle ou benzyle.

11. Composé comprenant au moins deux composés selon l'une des revendications 1 à 10, qui sont reliés ensemble par le biais d'un lieur *(linker),* le lieur étant en particulier une chaîne alkyle, un peptide, un oligonucléotide ou un oligomère, qui est constitué d'au moins trois motifs acide 8-amino-3,6-dioxaoctanoïque

12. Composition comprenant au moins un composé selon l'une des revendications précédentes.

13. Composition pharmaceutique comprenant au moins un composé ou une composition selon l'une des revendications 1 à 12, le cas échéant en combinaison avec au moins un véhicule, un solvant ou d'autres auxiliaires pharmaceutiques.

14. Utilisation d'un composé ou d'une composition selon l'une des revendications 1 à 12 ou d'une composition pharmaceutique selon la revendication 13 pour la préparation d'un médicament destiné au traitement des maladies virales, du cancer, des maladies inflammatoires, des maladies neurologiques, des maladies du tractus gastro-intestinal ou des maladies métaboliques, en particulier pour le traitement du VIH, du SIDA ou de l'hépatite, ou au traitement du cancer de la peau, du cancer du poumon, du cancer du foie, du cancer de la prostate, de la leucémie ou des tumeurs cérébrales, ou au traitement de l'asthme ou du psoriasis, ou au traitement de la maladie de Parkinson, ou au traitement du cancer de l'intestin, de la maladie de Crohn ou de l'obésité, ou au traitement de maladies qui sont liées à un taux de cholestérol élevé dans le sang.
